# EUROPEAN PATENT SPECIFICATION

(11) **EP 0 681 568 B1**
(45) Date of publication and mention of the grant of the patent: **26.07.2006**
(21) Application number: 94906685.6
(22) Date of filing: 28.01.1994
(51) Int. Cl.: C07D 205/08, C07D 305/14

(54) **PROCESS FOR PREPARATION OF TAXANE DERIVATIVES AND BETA-LACTAM INTERMEDIATES THEREFOR**
VERFAHREN ZUR HERSTELLUNG VON TAXANDERIVATEN UND BETA-LACTAM ZWISCHENPRODUKTE
PROCEDE DE PREPARATION DE DERIVES DE TAXANE ET INTERMEDIAIRES DE BETA-LACTAME UTILISES A CET EFFET

(30) Priority: 01.02.1993 US 11922
(43) Date of publication of application: 15.11.1995
(73) Proprietor: THE RESEARCH FOUNDATION OF STATE UNIVERSITY OF NEW YORK, NY 11794-0001 (US)
(72) Inventor: OJIMA, Iwao, Stony Brook, NY 11790 (US)
(74) Representative: Bentham, Stephen
(86) International application number: PCT/US1994/000669
(87) International publication number: WO 1994/018164

(56) References cited:
- EP-A- 0 400 971
- EP-A- 0 525 589
- WO-A-93/06079
- WO-A-93/06093
- WO-A-93/06094
- TETRAHEDRON, (INCL. TETRAHEDRON REPORTS) vol. 48, no. 34 , 1992 , OXFORD GB pages 6985 - 7012 I. OJIMA ET AL. 'New and efficient approaches to the semisynthesis of taxol and its C-13 side chain analogs by means of beta-lactam synthon method' cited in the application
- JOURNAL OF ORGANIC CHEMISTRY vol. 56, no. 5 , 1991 , EASTON US pages 1681 - 1683 I. OJIMA ET AL. 'Efficient and practical asymmetric synthesis of the taxol C-13 side chain, N-benzoyl-(2R,3S)-3-phenylisoserine, and its analogues via chiral 3-hydroxy-4-aryl-beta-lactams through chiral ester enolate-imine cyclocondensation' cited in the application

## Description

### FIELD OF THE INVENTION

The present invention relates to a process for the preparation of taxoid(s) including TAXOTERE and its analogs.

### BACKGROUND OF THE INVENTION

Taxol (I) is a complex diterpene which is currently considered the most exciting lead in cancer chemotherapy. Taxol possesses high cytotoxicity and strong antitumor activity against different cancers which have not been effectively treated by existing antitumor drugs. For example, taxol is currently in phase III clinical trials for advanced ovarian cancer, phase II for breast cancer, and phase I for lung cancers, colon cancer and acute leukemia.

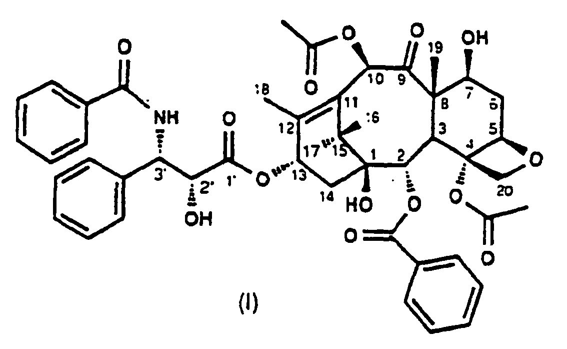

Although taxol is an extremely important "lead" in cancer chemotherapy, taxol has a problem with solubility in aqueous media, which may impose some serious limitation in its use. It is common for improved drugs to be derived from naturally occurring lead compounds. In fact, Frencn researchers, Potier, Guéritte-Voegelein, Guénard et al. have discovered that a modification of the C-13 side chain of taxol brought about a new anticancer agent which seems to have antitumor activity superior to taxol with better bioavailability. This synthetic compound was named "TAXOTÈRE (II)", which has t-butoxycarbonyl instead of benzoyl on the amino group of (2R,3S)-phenylisoserine moiety at the C-13 position and a hydroxyl group instead of an acetoxy group at C-10. [Colin, M. et al. Eur. Pat. Appl. EP253,738 (1988)]. Taxotère is currently in phase II clinical trial in both United States and Europe. TAXOTÈRE has been synthesized by a semisynthetic process, including a coupling of N-tert-butoxycarbonyl-(2R,3S)-3-phenylisoserine with 10-deacetylbaccatin III with proper protecting groups. (Denis, J.-N. recently reported (Commercon, A. et al., Tetrahedron Letters, 1992, 33 5185)).

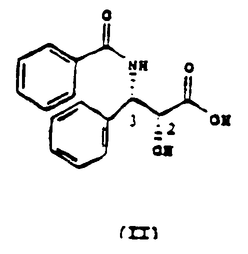

It is known that the C-13 side chain of taxol, i.e., N-benzoyl-(2R, 3S)-3-phenylisoserine (III) moiety, is crucial for the strong antitumor activity of taxol. (Senilh et al., C.R. Séancas Acad. Sci. Ser. 2 1984, 299, 1039; Guéritte-Voegelein et al., Tetrahedron, 1986, 42, 4451, and Mangatal et al., Tetrahedron, 1989, 45, 4177; Gueritte-Voegelein et al. J. Med. Chem. 1991, 34, 992; and Swindell et al., J. Med. Chem. 1992, 35, 145; Mathew, A.E. et al., J. Med. Chem. 1992, 35, 145). Moreover, some modification of the C-13 side chain can provide a new series of taxol analogs which may have higher potency, better bioavailability and less unwanted toxicity, as exemplified by the discovery of TAXOTÈRE (II).

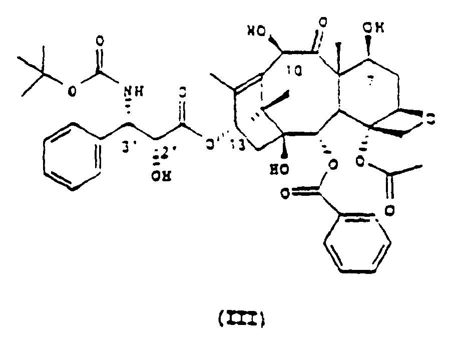

Accordingly, the development of an efficient method which can be applied to various analogs of taxol and TAXOTÈRE and analogs thereof, i.e., a method having flexibility and wide applicability, is extremely important and of current demand. It has been shown that such a new and efficient method with flexibility can be developed by using enantiomerically pure β-lactams as key-intermediates [Ojima, I. et al., J. Org. Chem., 1991, 56, 1681; Ojima et al., Tetrahedron, 1992, 48, 6985; Holton, R.A., Eur. Patent Appl. EP 400,971 (1990)].

Lithium chiral ester enolate-imine cyclocondensation strategy has been applied to the asymmetric synthesis of the side chain of taxol via a (3*R*,4*S*)-3-hydroxy-4-phenylazotidin-2-one (IV) as the key-intermediate. (Ojima, I. et al., J. Org. Chem., 1991, 56, 1681; Ojima et al., Tetrahedron, 1992, 48, 6985)

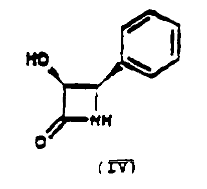

Based on this protocol, the side chain can be obtained in 3 steps in high yield with virtually 100% e.e. (Ojima, I. et al. J. Org. Chem. 1991 56, 1681). Recently, it was found that 1-benzoyl-(3*R*,4*S*)-3-(1-ethoxyethoxy)-4-phenylazetidin-2-one (V), readily derived from the hydroxy-β-lactam (IV), served as the key-intermediate for the synthesis of taxol [Holton, R.A. Eur. Pat. Appl. EP 400,971 (1990)]. Therefore, this β-lactam intermediate serves as the key-intermediate for both coupling methods.

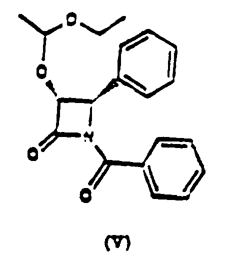

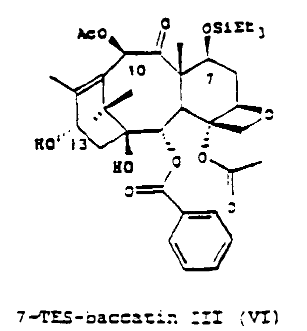

In the published European application to Holton (hereinafter Holton), the β-lactam intermediate (V) was obtained through tedious optical resolution of the racemic cis-3-hydroxy-3-lactam. According to Holton's procedure, the coupling of the β-lactam (V) with 7-triethylsilylbaccatin III (VI) (7-TES-baccatin III) proceeds at 25°C in the presence of dimethylaminopyridine (DMAP) and pyridine for 12 hours to give protected taxol in 92% yield, which was deprotected with 0.5% hydrochloric acid in ethanol at 0°C to afford taxol in ca. 90% yield.

However, the Holton procedure did not work at all when 1-tert-butoxycarbonyl-(3*R*,4*S*)-3-(1-ethoxylethoxy)-4-phenylazetidin-2-one (VII) was used for the attempted synthesis of TAXOTÈRE (II) by the present inventors.

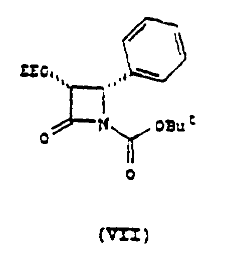

It is believed that this may be due to the lack of reactivity of the 1-tert-butoxycarbonyl-β-lactam (VII) toward the C-13 hydroxyl group of a protected baccatin III (VI or VIII) under the conditions used by Holton. The lack of reactivity may be ascribed to the substantially weaker electron-withdrawing ability of tert-butoxycarbonyl group than that of benzoyl group.

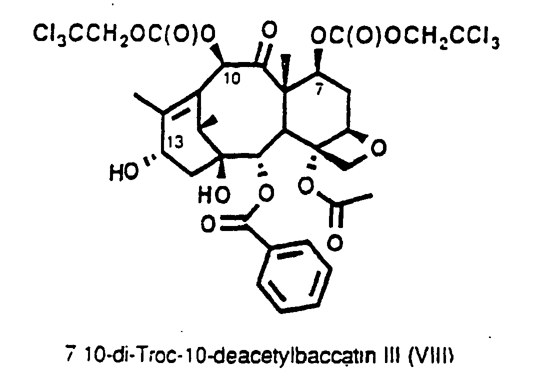

WO-A-93/06093 discloses a process for the preparation of 10-desacetoxy taxol derivatives by reaction of β-lactam intermediates with taxol derivatives but was published after the priority date of the present application.

EP-0 525 589 discloses a process for the preparation of (3R, 4S)-3-hydroxy-4-phenyl-2-azetidinone derivatives which are useful intermediates which are useful in the synthesis of taxol from baccatin III but was published after the priority date of the present application.

WO-A-93/06094 and WO-A-93/06079 disclose a process for the preparation of derivatives by reaction of β-lactam intermediates with baccatin III derivatives but were published after the priority date of the present application. Neither discloses the use of certain soluble bases in the reaction.

Therefore, it was an objective of the present invention to develop a new method which can achieve the coupling of the 1-tert-butoxycarbonyl-β-lactam (VII) with the protected baccatin III (VIII) for the synthesis of TAXOTÈRE (II).

### SUMMARY OF THE INVENTION

A β-lactam of the formula (IX) 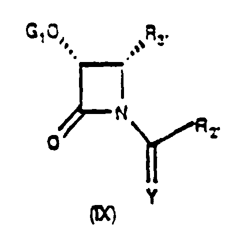 in which
R_{2'} represents an RO-, RS- or RR'N- in which R represents an unsubstituted or substituted straight chain or branched alkyl, alkenyl or alkynyl, cycloalkyl, heterocycloalkyl, cycloalkenyl, heterocycloalkenyl, carbocyclic aryl or heteroaryl, wherein substituents bearing one or more active hydrogens such as hydroxyl, amino, marcapto and carboxyl groups are protected; R' is a hydrogen or R as defined above; R and R' can be connected to form a cyclic structure; Examples of R_{2'} include methoxy, ethoxy, isopropoxy, tert-butoxy, neopentyloxy, cyclohexyloxy, allyloxy, propargyloxy, adamantyloxy, phenyoxy, 4-methoxyphenoxy, 2-fluorophenoxy, 4-methoxycarbonylphenoxy, methylthio, ethylthio, isopropylthio, tert-butylthio, neopentylthio, cyclohexylthio, phenylthio, 3,4-dimethoxyphenylthio, methylamino, ethylamino, isopropylamino, tert-butylamino, neopentylamino, cyclohexylamino, dimethylamino, pyrrolidino, piperidino and morpholino group.
R_{3'} represents an unsubstituted or substituted straight chain or branched alkyl, alkenyl or alkynyl radical, an unsubstituted or substituted cycloalkyl, or cycloalkenyl radical, an unsubstituted or substituted aryl radical wherein substituents bearing one or more active hydrogens such as hydroxy, amino, mercapto and carboxyl groups are protected; Examples of R_{3'} include phenyl, 4-methoxyphenyl, 3,4-dimethoxylphenyl, 4-fluorophenyl, 4-trifluoromethylphenyl, 4-chlorophenyl, 4-bromophenyl, naphthyl, cyclohexyl, cyclohexylmethyl, 2-phenylethenyl, 2-phenylethyl, benzyl, neopentyl, tert-butyl, isobutyl, isopropyl, allyl and propargyl;
G₁ represents a hydrogen or hydroxyl protecting group such as methoxymethyl (MOM), methoxylethyl (MEM), 1-ethoxyethyl (EE) benzyloxymethyl, (β-trimethylsilylethoxyl)methyl, tetrahydropyranyl, 2,2,2-trichloroethoxycarbonyl (Troc), tert-butoxycarbonyl (t-BOC), 9-fluorenylmethoxycarbonyl (Fmoc), 2,2,2-trichloroethoxymethyl, trimethylsilyl, triethylsilyl, dimethylethylsilyl, dimethyl(t-butyl)silyl, diethylmethylsilyl, dimethylphenylsilyl and diphenylmethylsilyl;
Y is oxygen or sulfur is useful in the preparation of taxoids.

The present inventor investigated the β-lactam coupling reaction with protected Baccatin III in detail and found that the coupling could be achieved by increasing the nucleophilicity of the 13-hydroxyl group of a protected baccatin III (VI or VIII) through transformation of the hydroxyl group to the corresponding alkali metal alkoxide. Such a C-13 alkali metal alkoxide of a baccatin III was readily generated by reacting the baccatin III (VI or VIII) with an alkali metal base. This finding is the basis of the present invention. The method of the present invention not only enables the coupling of the β-lactam (VII) and its derivatives and analogs with a protected baccatin III, but also requires only a stoichiometric amount of the β-lactams. The latter makes a sharp contrast with the Holton procedure for taxol synthesis which needs 5-6 equivalents of the more reactive β-lactam (V). Moreover, the coupling reactions of the present invention proceed very smoothly and complete typically within 30 minutes at -30°C - 0°C.

The present invention provides a process for the preparation of taxane derivatives of the formula (X) 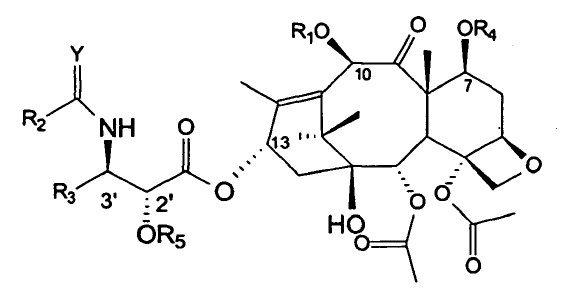 in which
R₁ represents a hydrogen or an acyl or an alkyl or an alkenyl or an alkynyl or a carbocyclic aryl or a heteroaryl radical or a hydroxyl protecting group;
R₂ represents an RO-, RS- or RR'N- in which R represents a straight chain or branched alkyl, alkenyl or alkynyl, cycloalkyl, heterocycloalkyl, cycloalkenyl, heterocycloalkenyl, carbocyclic aryl or heteroaryl radical, these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals, the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms; R' is hydrogen or R defined above; R and R' can be connected to form a cyclic structure;
Y is oxygen or sulfur;
R₃ represents a straight chain or branched alkyl, alkenyl or alkynyl, cycloalkyl, cycloalkenyl or carbocyclic aryl radical, these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl radicals, the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms;
R₄ represents hydrogen or an acyl radical or an unsubstituted straight chain or branched alkyl, alkenyl or alkynyl radical, an unsubstituted cycloalkyl, heterocycloalkyl, cycloalkenyl or heterocycloalkenyl radical, an unsubstituted aryl or heteroaryl radical, or a hydroxyl group protecting group;
R₅ represents hydrogen or an acyl radical or an unsubstituted straight chain or branched alkyl, alkenyl, or alkynyl radical, an unsubstituted cycloalkyl, heterocycloalkyl, cycloalkenyl or heterocycloalkenyl radical, an unsubstituted aryl or heteroaryl radical, or a hydroxyl protecting group;
which process comprises preparing a baccatin III derivative of the formula: 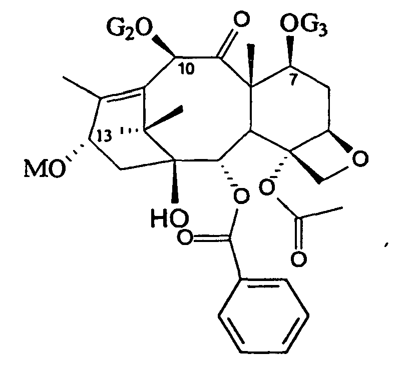 in which M is an alkali metal atom (ion);
G₂ represents a hydroxyl protecting group or an acyl radical or an unsubstituted straight chain or branched alkyl, alkenyl, or alkynyl radical, an unsubstituted cycloalkyl, heterocycloalkyl, cycloalkenyl or heterocycloalkenyl, radical, or an unsubstituted aryl or heteroaryl radical; and
G₃ represents a hydroxyl protecting group or an acyl radical or an unsubstituted straight chain or branched alkyl, alkenyl or alkynyl radical, an unsubstituted cycloalkyl, heterocycloalkyl, cycloalkenyl, or heterocycloalkenyl radical, or an unsubstituted aryl or heteroaryl radical,
by reacting a protected baccatin III of formula: 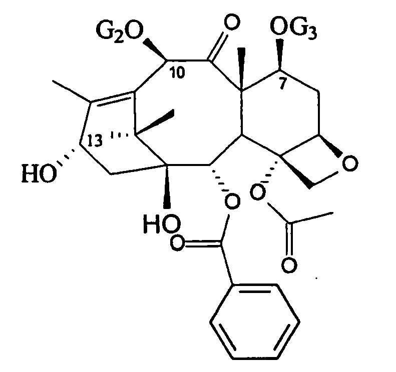 in which G₂ and G₃ are as defined above with a soluble alkali metal base which base is sodium hexamethyldisilazide, potassium hexamethyldisilazide, lithium hexamethyldisilazide, sodium diisopropylamide or potassium diisopropylamide, and then reacting a β-lactam of the formula: 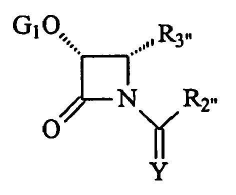 in which
Y is defined above: G₁ represents a hydroxyl protecting group:
R_{2"} represents a radical R₂ defined above or a protected R₂ whenever R₂ includes one or more active hydrogens:
R_{3"} represents a radical R₃ defined above or a protected R₃ whenever R₃ includes one or more active hydrogens:
with the baccatin III derivative.

Groups including active hydrogens in the definitions of R₂ and R₃ are, for example, hydroxyl, amino, mercapto and carboxyl groups.

In one embodiment of the process according to the invention R² represents a radical RO-, RS-, or RR'N- in which R represents a straight chain or branched alkyl radical containing 1 to 10 carbon atoms, a straight chain or branched alkenyl radical containing 2 to 10 carbon atoms, or a straight chain or branched alkynyl radical containing 2 to 10 carbon atoms, a cycloalkyl radical containing 3 to 10 carbon atoms, a heterocycloalkyl radical containing 3 to 10 carbon atoms, a cycloalkenyl radical containing 3 to 10 carbon atoms, a heterocycloalkenyl radical containing 3 to 10 carbon atoms, a polycycloalkyl radical containing 6 to 20 carbon atoms, an aryl radical containing 6 to 20 carbons, a heteroaryl radical containing 3 to 15 carbon atoms; these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms; R' is hydrogen or R defined above; R and R' can form a cyclic structure which contains 2-10 carbon atoms;
R₃ represents a straight chain or branched alkyl radical containing 1 to 10 carbon atoms, a straight chain or branched alkenyl radical containing 2 to 10 carbon atoms, or a straight chain or branched alkynyl radical containing 2 to 10 carbon atoms, a cycloalkyl radical containing 3 to 10 carbon atoms, a cycloalkenyl radical containing 3 to 10 carbon atoms, a polycycloalkyl radical containing 6 to 20 carbon atoms, an aryl radical containing 6 to 10 carbon atoms; these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals, the alkyl portion of which contains 1 to 15 carbon atoms, aryloxycarbonyl, the aryl portion of which contains 6 to 20 carbon atoms, or heteroaryloxycarbonyl the heteroaryl portion of which contains 3 to 15 carbon atoms;
R_{2"} represents a radical R₂ defined above or a protected R₂ whenever R₂ includes one or more active hydrogens;
R_{3"} represents a radical R₃ defined above or a protected R₃ whenever R₃ includes one or more active hydrogens.

According to another embodiment of the precess according to the invention R₂ represents an RO-, RS- or RR'N- in which R is an unsubstituted or substituted radical selected from methyl, ethyl, propyl, isopropyl, butyl, isobutyl, tert-butyl, pentyl, isopentyl, neopentyl, hexyl, isohexyl, heptyl, isoheptyl, octyl, isooctyl, cyclopropyl, cyclobutyl, cyclopentyl, cyclohexyl, cycloheptyl, cyclooctyl, adamantyl, vinyl, allyl, phenyl, naphthyl, furyl, pyrrolyl, pyridyl, cyclopentenyl, cyclohexenyl, cycloheptenyl, oxiranyl, tetrahydrofuryl, pyrrolidinyl, piperidinyl, tetrahydropyranyl, dihydrofuryl, dihydropyrrolyl, dihydropyranyl, dihydropyridyl; R is hydrogen or R defined above; cyclic RR'N- radical is aziridino, azetidino, pyrrolidino, piperidino or morpholino group;
R₃ is an unsubstituted or substituted radical selected from methyl, ethyl, propyl, isopropyl, butyl, isobutyl, tert-butyl, pentyl, isopentyl, neopentyl, hexyl, isohexyl, heptyl, isoheptyl, octyl, isooctyl, cyclohexylinethyl, cyclohexylethyl, benzyl, phenylethyl, cyclopropyl, cyclobutyl, cyclopentyl, cyclohexyl, cycloheptyl, cyclooctyl, adamantyl, vinyl, allyl, ethynyl, propargyl, phenyl, naphthyl, cyclopentenyl, cyclohexenyl and cycloheptenyl;
R_{2"} represents a radical R₂ defined above or a protected R₂ wherever R₂ includes one or more active hydrogens;
R_{3"} represents a radical R₃ defined above or a protected R₃ wherever R₃ includes one or more active hydrogens;
G₁ represents a group protecting the hydroxyl function selected from methoxylmethyl (MOM), methoxyethyl (MEM), 1-ethoxyethyl (EE), benzyloxymethyl, (β-trimethylsilyl-ethoxyl)-methyl, tetrahydropyranyl, 2,2,2-trichloroethoxylcarbonyl (Troc), benzyloxycarbonyl (CBZ), tert-butoxycarbonyl (t-BOC), 9-fluorenylmethoxycarbonyl (Fmoc), 2,2,2-trichloroethoxymethyl, trimethylsilyl, triethylsilyl, tripropylsilyl, dimethylethylsilyl, dimethyl (t-butyl)silyl, diethylmethylsilyl, dimethylphenylsilyl and diphenylmethylsilyl, acetyl, chloroacetyl, dichloroacetyl, trichloroacetyl and trifluoroacetyl;
G₂ represents an acetyl or a 2,2,2-trichloroethoxycarbonyl (Troc) group;
G₃ represents a 2,2,2-trichloroethoxycarbonyl (Troc) or silyl group selected from trimethylsilyl, triethylsilyl, tripropylsilyl, dimethylethylsilyl, dimethylphenylsilyl, dimethyl(t-butyl)silyl, diethylmethylsilyl and diphenylmethylsilyl.

In one embodiment of the process of the invention M is sodium or potassium.

In another embodiment R₁ is hydrogen, an acetyl or a trichloroethoxycarbonyl (Troc); R₄ is a hydrogen, a triethylsilyl or a trichloroethoxycarbonyl (Troc); R₅ is a hydrogen, a triethylsilyl or ethoxyethyl.

In another embodiment R₂ represents RO- in which R is a methyl, ethyl, propyl, isopropyl, butyl, isobutyl, tert-butyl, neopentyl, cyclohexyl, phenyl, benzyl or 9-fluoroenylmethyl; R₃ is phenyl, tolyl, 4-methoxyphenyl, 3,4-dimethoxyphenyl, 4-fluorophenyl, 4-trifluoromethylphenyl, 1-naphthyl, 2-naphthyl or 2-phenylethenyl; R₅ is a hydrogen.

In another embodiment R₂ is a methylamino, ethylamino, propylamino, isopropylamino, butylamino, isobutylamino, tert-butylamino, neopentylamino, cyclohexylamino, phenylamino or benzylamino, dimethylamino or morpholino group; R₅ is a hydrogen.

In another embodiment R₁ is a hydrogen or an acetyl; R₂ (=R₂") is tert-butoxy or tert-butylamino; R₃ (=R₃") is a phenyl; Y is oxygen; R₄ is a hydrogen; R₅ is a hydrogen; G₁ is an ethoxyethyl, triethylsilyl or trichloroethoxycarbonyl (Troc); M is sodium or potassium.

The β-lactams of the formula (IX) herein above are synthesized by modifying the β-lactams of the formula (XI) 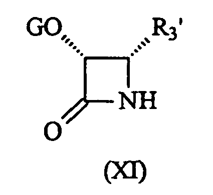 wherein G is a hydroxyl protecting group such as triisopropylsilyl (TIPS) and dimethyl (tert-butyl) silyl (TBDMS), and R₃' has been defined hereinabove.

The β-lactams (XI) are readily prepared by using the chiral enolate - imine cyclocondensation method which has been developed in the present inventor's laboratory as shown in Scheme 1 (Ojima, I. et al., Tetrahedron, 1992, 48, 6985; Ojima, I. et al., J. Org. Chem. 1991, 56, 1681). In this preparation the β-lactams (XI) with extremely high enantiomeric purities are obtained in high yields. In Scheme, R* is a chiral auxiliary moiety which is (-)- trans-2-phenyl-1-cyclohexyl, TMS is a trimethylsilyl radical, and base is lithium disopropylamide or lithium hexamethyldisilazide; G and R₃' have been defined hereinabove.

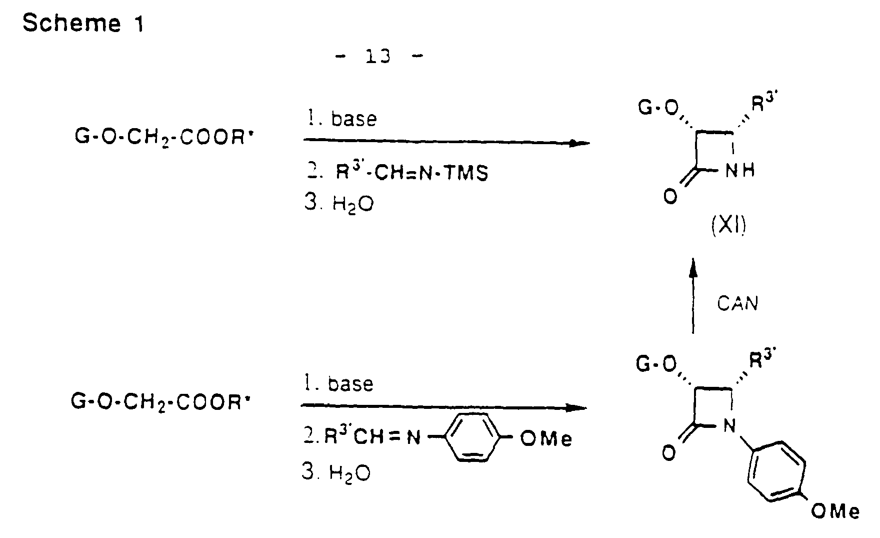

The β-lactams (XI) are converted to the 3-hydroxy-β-lactams (XII), followed by protection with ethoxyethyl group (EE) to give the β-lactams (XIII). The β-lactams (XIII) are reacted with chloroformates or formic anhydrides or thiocholorformates or thioformic anhydrides in the presence of a base to yield the β-lactams (XIV) (or thioanalogs thereof) which are used for the coupling with protected 10-deacetylbaccatin III to produce TAXOTÈRE and its analogs. The β-lactams (XIV) are deprotected under weakly acidic conditions to afford the β-lactams (XV) which can serve as very useful intermediates to the β-lactams (XVI) bearing a variety of protecting groups (G₁) at the C-3 position of β-lactam skeleton. The β-lactams (XVI) can also be used for the coupling with a protected 10-deacetylbaccatin III to produce Taxotère and its analogs after deprotection.

In a similar manner, the β-lactams (XVII) are prepared by reacting the β-lactams (XIII) with isocyanates or isothiocyanates in the presence of a base which can be used for the protection of other potent anticancer agents of formula (X) in which R₂ represents RRN-. The β-lactams (XVII) are deprotected under weakly acidic conditions to give the β-lactams (XVIII) which can serve as very useful intermediates to a variety of protected 3-hydroxyl-β-lactams (XIX). The β-lactams (XVII and XIX) can also be used for the coupling with a protected 10-deacetylbaccatin III to yield a compound of formula (X) in which R₂ represents RR'N- after deprotection.

In a manner similar to that described above, the β-lactams (XX) are prepared by reacting the β-lactams (XIII) with N,N-disubstituted carbamoyl halides in the presence of a base. The β-lactams (XX) are deprotected under weakly acidic conditions to give the 3-hydroxy-β-lactams (XXI), which can serve as very useful intermediates to various protected 3-hydroxy-β-lactams (XXII). The β-lactams (XX and XXII) can readily be used for the coupling with a protected baccatin III to afford a compound of formula (X) after deprotection.

The transformations described above are illustrated in Scheme 2. In Scheme 2, X represents a leaving group such as fluoride, chloride, bromide, iodide, tosylate, mesylate and trifluoromesylate. G₁ represents a group protecting the hydroxyl function selected from methoxylmethyl (MOM), methoxyethyl (MEM), 1-ethoxyethyl (EE), benzyloxymethyl, (β-trimethylsilylethoxyl) methyl, tetrahydropyranyl, 2,2,2-trichloroethoxylcarbonyl (TROC:), benzyloxycarbonyl (CBZ), tert-butoxycarbonyl (t-BOC), 9-fluorenyl methoxycarbonyl (FMOC) 2,2,2-trichloroethoxymethyl, trimethyl silyl, dimethyl(t-butyl)silyl, diethylmethylsilyl, dimethyl phenylsilyl and diphenylmethylsilyl, acetyl, chloroacetyl, dichloroacetyl, trichloroacetyl and trifluoroacetyl. R^{2'}, R^{3'}, R, and R' are defined hereinabove.

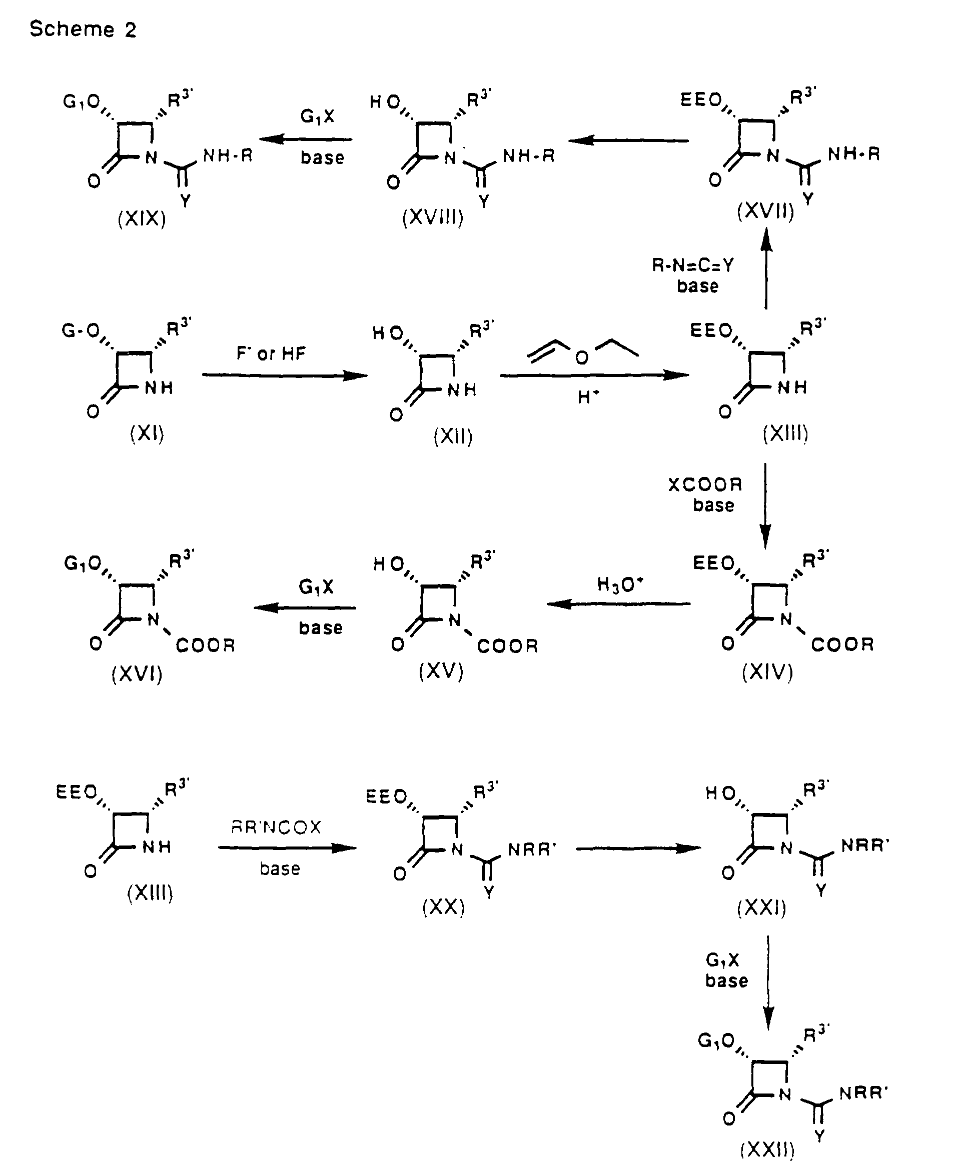

The β-lactams (XIV) and (XVI) are readily used for the coupling with protected baccatin IIIs in the presence of base, followed by deprotection to give TAXOTÈRE and its analogs in high yields (Scheme 3). In a similar manner, the β-lactams (XVII and XIX) with protection of -NH- moiety) and the β-lactams (XX and XXII) can be used for the coupling with protected baccatin IIIs, followed by deprotection to give a compound of formula (X) in which R₂ represents RR^{1'}N- (Scheme 3).

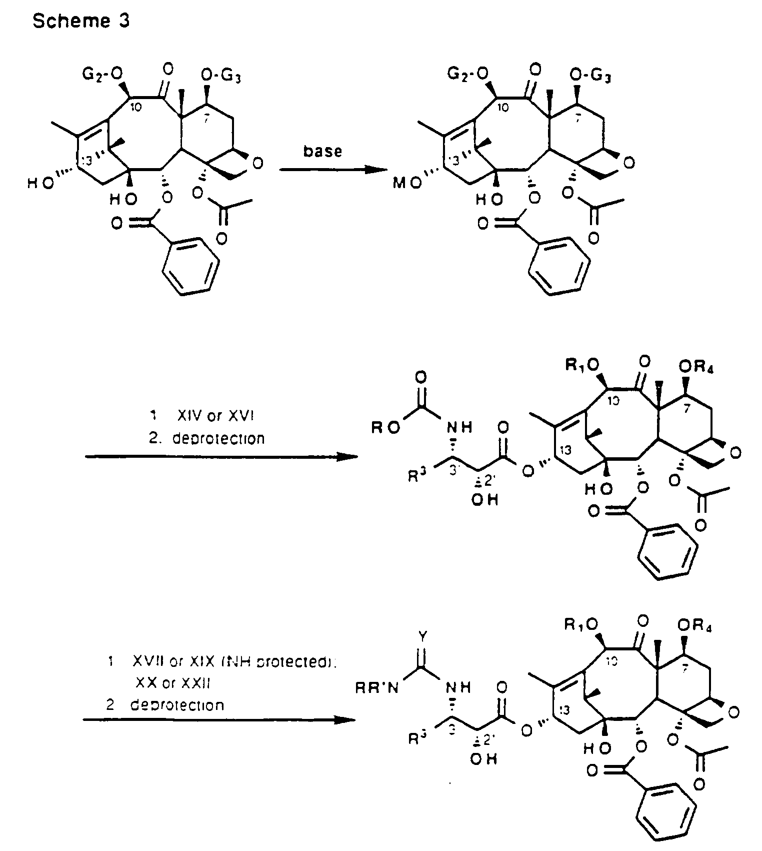

G₂ and G₃ represents an hydroxyl protecting group or an acyl radical or an unsubstituted or substituted straight chain or branched alkyl, alkenyl radical, an unsubstituted or substituted cycloalkyl, heterocycloalkyl, cycloalkenyl or heterocycloalkenyl radical, an unsubstituted or substituted carbocyclic aryl or heteroaryl radical.

When G₂ and G₃ are hydroxyl protecting groups G₁ defined above and 1-ethoxyethoxyl (EE), these protecting groups can be attached to the hydroxyl groups of 10-deacetylbaccatin III and its analogs by methods which are generally known to those skilled in the art.

The coupling reaction of the protected baccatin III and the β-lactam is carried out via the alkali metal alkoxide of the protected baccatin III at the C-13 hydroxyl group. The alkoxide can readily be generated by reacting the protected baccatin III with a soluble alkali metal base such as sodium hexamethyldisilazide, potassium hexamethyldisilazide, lithium hexamethyldisilazide, sodium diisopropylamide, potassium diisopropylamide or lithium diisopropylamide generally in a dry nonprotic organic solvent such as tetrahydrofuran (THF), dioxane, ether, dimethoxyethane (DME), diglyme, dimethylformamide (DMF), mixtures of these solvents with hexane, toluene, a xylene, in a preferred temperature range from about -100°C. to about 50°C, more preferably at about -78°C to about 25 °C. This reaction is preferably carried out under inert atmosphere such as nitrogen and argon. The amount of the base used for the reaction is preferably approximately equivalent to the amount of the protected baccatin III. The use of a slight excess of the base does not adversely affect the reaction.

The coupling reaction of the metal alkoxide of the protected baccatin III thus generated with the β-lactam is typically carried out by adding the solution of the β-lactam in a dry organic solvent exemplified above in a preferred temperature range from about -100°C to 50°C, more preferably at about -35°C to 25 °C. The mixture of reactants is stirred for 15 minutes to 24 hours and the progress and the completion of the reaction is monitored by thin layer chromatography (TLC), for example. When the limiting reactant is completely consumed the reaction is quenched by addition of a brine. The crude reaction mixture is worked up using the standard isolation procedures which are generally known to those skilled in the art to give the corresponding protected taxoid. The proportion of the β-lactam and the protected baccatin III is in a range from 2:1 to 1:2, more preferably approximately 1:1 for purposes of economy and efficiency, but the ratio is not critical for the reaction.

The protecting gruops, EE, G₁, G₂ and G₃, can then be removed by using the standard procedures which are generally known to those skilled in the art to give the desired taxane derivatives. For example, EE and triethylsilyl groups can be removed with 0.5 N HCl at room temperature for 36 h, and Troc group can be removed with zinc and acetic acid in methanol at 60°C for 1 hour without disturbing the other functional groups and the skeleton of the taxoid. The following non-limiting examples are illustrative of the present invention.

### Examples 1-2

**(3*R*,4*S*)-3-Triisopropylsilyloxy-4-phenyl-2-azetidinone (1a):** to a solution of 645 mL (4.6 mmol) of diisopropylamine in 10 mL of THF, was added 1.85 mL (4.6 mmol, 2.5M) of n-BuLi at 0°C. The solution was stirred 1 h at 0°C followed by the addition of 1.5 g (3.8 mmol) of (-) TIPS ester in 15 mL of THF over a 1 h period (using a cannula) at -78°C. The reaction was stirred 2 h at this temperature followed by the addition of 817 mg (4.6 mmol) of N-TMS benzaldimine in 15 mL of THF over a 2 h period at -95°C. The reaction was stirred overnight at this temperature and allowed to slowly warm up at room temperature. The reaction was quenched by addition of sat. NH₄Cl. The aqueous layer was extracted with ether. The organic layer was washed with 3% HCl and brine, dried over MgSO₄ and concentrated. The crude oil was purified by chromatography on silica gel using 1:5 EtAco/hexanes to give 1.03 g (84%) of β-lactam as a white solid: Mp 76-77°C; [α]D²⁰ +52.7° (C 1.00, CHCl₃); ¹H NMR (300 MHz, CDCl₃) δ 0.86-0.93 (m, 21H), 4.81 (d, J = 4.7 Hz, 1H), 5.17 (dd, J = 4.7, 2.6 Hz, 1H), 6.18 (bs, 1H), 7.17-7.35 (m, 5H); ¹³C NMR (75 MHz, CDCl₃ δ 11.8, 17.4, 17.5, 59.6, 79.9, 127.9, 128.0, 128.1, 136.4, 170.0; IR (KBr) 3234, 2946-2866, 1760, 1458 cm⁻¹. Anal. Calcd for C₁₈H₂₉NO₂Si: C 67.66%, H 9.15%, N 4.38%. Found: C 67.64%, H 9.25%, N 4.44%.

In the same manner, β-lactam **1b** was obtained in good yield.
**(3*R*,4*S*)-3-Triisopropylsilyloxy-4-(2-phenylethenyl)-2-azetidinone (1b):** 72%; colorless liquid; ₁H NMR (300 MHz, CDCl₃) δ 0.98-1.02 (m, 21H), 4.36 (dd, J = 4.6, 8.3 Hz, 1H), 5.09 (dd, J = 2.3, 4.6 Hz, 1H), 6.29 (dd, J = 8.3, 16.0 Hz, 1H), 6.59 (d, J = 16.0 Hz, 1H), 6.83, (bs, 1H), 7.23-7.39 (m, 5H); NMR (75 MHz, CDCl₃) δ 11.79, 17.61, 17.66, 58.34, 79.86, 126.05, 126.45, 127.90, 128.56, 134.41, 136.30, 169.69; IR (neat) 3262, 3032, 2944, 2865, 1748, 1672, 1623 cm⁻¹. Anal. Calcd for C₂₀H₃₁NO₂Si: C, 69.52; H, 9.04; N, 4.05. Found: C, 69.75; H, 9.02; N, 3.89.

### Examples 3-4

To a solution of 2.51 mmol of diisopropylamine in 15 mL of THF was added 2.51 mL of n-butyllithium (2.5M in THF) at -10°C. After 30 min, the lithium diisopropylamide (LDA) was generated and the solution was cooled to -95°C. A solution of 2.17 mmol of chiral ester in 5 mL of THF was added. After 1 hr, a solution of 2.5 mmol of the appropriate imine in 3mL of THF was added. The mixture was stirred at -95°C overnight, and the progress of the reaction was monitored by TLC or ¹H NMR. The reaction was quenched with sat. NH₄Cl and THF was removed using a rotary evaporator. Ether (10 mL) was added and the aqueous layer was extracted with ether (10 mL x3). Drying and removal of the solvent gave the crude product which was purified by silica gel column chromatography (hexane/ethyl acetate=10:1) to afford the corresponding pure β-lactam. The enantimeric excess was determined by HPLC using a CHIRALCEL OD column using n-hexane/i-PrOH (90/10) as the eluent.
**(3*R*,4*S*)-4-(2-Methylpropyl)-1-(4-methoxyphenyl)-3-triisopropylsilyloxy-2-azetidinone (2a):** 87%; pale yellow solid; mp 59-60°C; [α]D²⁰ +60.46° (c 1.26, CHCl₃); ¹H NMR (300 MHz, CDCl₃) δ 0.96 (d, J = 6.4 Hz, 3H), 1.03 (d, J = 6.4 Hz, 3H), 1.10-1.30 (m, 21H), 1.60-1.68 (m, 1H), 1.70-1.92 (m, 2H), 3.75 (s, 3H), 4.16-4.22 (m, 1H), 5.06 (d, J = 5.1 Hz, 1H), 6.86 (d, J = 9.0 Hz, 2H), 7.32 (d, J = 9.0 Hz, 2H); ¹³C NMR (75 MHz, CDCl₃) δ 12.34, 17.82, 17.91, 22.18, 23.37, 25.34, 35.89, 55.50, 57.33, 76.34, 114.52, 118.73, 131.00, 156.29, 165.58; IR (KBr) 2946, 1742, 1513, 1458, 1249 cm⁻¹. Anal. Calcd for C₂₃H₃₉NO₃Si: C, 68.10; H, 9.70; N, 3.45. Found: C, 68.26; H, 9.85; N, 3.35.
**(3*R*,4*S*)-4-(Cyclohexylmethyl)-1-(4-methoxyphenyl)-3-triisophropylsilyloxy-2-azetidinone (2b):** 83%; low melting point solid; [α]D²⁰ +43.7° (c 0.92, CHCl₃); ¹H NMR (300 MHz, CDCl₃) δ 0.85-1.95 (m, 34H), 3.78 (s, 3H), 4.19-4.25 (m, 1H), 5.05 (d, J = 5.1 Hz, 1H), 6.86 (d, J = 9.0 Hz, 2H), 7.32 (d, J = 9.0 Hz, 2H); ¹³C NMR (75 MHz, CDC₁₃) δ 12.15, 17.76, 17.83, 26.12, 26.22, 26.47, 32.84, 34.22, 34.51, 55.36, 56.41, 76.13, 114.30, 118.45, 130.81, 155.99, 165.55; IR (neat) 2925-2865, 1749, 1513, 1464, 1448, 1389, 1246, 1174, 1145, 1128, 939, 882, 828, 684 cm⁻¹. Anal. Calcd for C₂₆H₄₃NO₃Si: C, 70.06; H, 9.72; N, 3.14. Found: C, 69.91; H, 9.71; N, 3.02.

### Examples 5-6

To a solution of 0.24 mmol of 1-(4-methoxyphenyl)-β-lactam in CH₃CN (20 mL) was added 0.65 mmol of CAN in 10 mL CH₃CN and 20 mL of water in 20 min at -15°C. After stirring for 1 hr, it was diluted with water (20 mL), and the mixture was then extracted with ethyl acetate (15 mL x2). The combined organic layer was washed with NaHSO₃ water (7 mL), 5% (10 mL x 2), 5% Na₂CO₃ (10 mL) and brine (5 mL) in sequence. Drying, removal of the solvent in vacuo followed by decolorization with activated charcoal afforded the crude product. It was further purified by silica gel column chromatography (hexanes/ethyl acetate, 3/1) to furnish N-deprotected β-lactam.
**(3*R*,4*S*)-4-(2-Hethylpropyl)-3-triisopropylsilyloxy-2-azetidinone (1c):** 83%; yellow oil; [α]D²⁰+35.45° (c 1.33, CHCl₃); ¹H NMR (300 MHz, CDCl₃) δ 0.93 (d, J = 6.6 Hz, 3H), 0.96 (d, J = 6.6 Hz, 3H), 1.05-1.25 (m, 22H), 1.52 (M, 1H), 1.67 (m, 1H), 3.78 (m, 1H), 4.96 (dd, J = 4.8, 2.4 Hz, 1H), 6.02 (bs, 1H); ¹³C NMR (75MHz, CDCl₃) δ 12.12, 17.72, 17.80, 22.29, 23.08, 25.35, 39.08, 54.45, 78.04, 170.00; IR (neat) 3238, 1759, 1465, 1184 cm⁻¹. Anal. Calcd for C₁₆H₃₃NO₂Si: C, 64.16; H, 11.1; N, 4.68. Found: C, 64.17; H, 10.96; N, 4.47.
**(3*R*,4*S*)-4-(Cyclohexylmethyl)-3-triisopropylsilyloxy-2-azetidinone (1d):** 85%; yellow oil; [α]D²⁰+12.44° (c 1.46, CHCl₃); ¹H NMR (300 MHz, CDCl₃) δ 0.97-1.25 (m, 32H), 1.40-1.70 (m, 2H), 3.80 (dt, J = 8.4, 4.8 Hz, 1H), 4.95 (dd, J = 4.8, 2.4 Hz, 1H), 6.05 (bs, 1H); ¹³C NMR (75 MHz, CDCl₃) δ 12.06, 17.77, 17.82, 26.16, 26.25, 26.46, 33.15, 33.82, 34.85, 37.72, 53.89, 77.98, 169.98; IR (neat) 3238, 1759, 1465, 1184 cm¹. Anal. Calcd for C₁₉H₃₇NO₂Si: C, 67.20; H, 10.98; N, 4.12. Found: C, 67.40; H, 10.79; N, 3.98.

### Examples 7-11

To a solution of 2.6 mmol of 3-triisopropylsilyloxy-4-substituted-2-azetidinone in 20 mL of THF, was added at room temperature 3.1 mmol (1M in THF) of NBu₄F. After 5 h, the solvent was evaporated and the crude oil was directly purified by chromatography on silica gel using 5:1 EtAcO/hexanes to afford of 3-hydroxy-4-substituted-2-azetidinone:
**(3*R*,4*S*)-3-Hydroxy-4-phenyl-2-azetidinone (3a):** 100%; white solid; mp 189-190°C; [α]D²⁰ +181.6° (c 0.5, CH₃OH); ¹H NMR (300 MHz, CD₃OD) δ 4.84 (d, J = 4.7 Hz, 1H), 5.04 (d, J = 4.7 Hz, 1H), 7.25-7.35 (m, 5H); IR (KBr) 3373, 3252, 1732, 1494 cm⁻¹. Anal. Calcd for C₉H₉NO₂: C 66.25%, H 5.56%, N 8.58%. Found: C 66.42%, H 5.74%, N 8.62%.
**(3*R*,4*S*)-3-Hydroxy-4-(2-phenylethenyl)-2-azetidinone (3b)**: 82%; white solid; mp 143-144°C; [α]D²⁰ +21.9° (c 1.05, MeOH); ¹H NMR (300 MHz, CD₃OD) δ 4.35 (ddd, J = 0.8, 4.7, 7.7 Hz, 1H), 4.93 (d, J = 4.7 Hz, 1H), 6.28 (dd, J = 7.7, 16.0 Hz, 1H), 7.18-7.43 (m, 5H); ¹³C NMR (75 MHz, CD₃OD) δ 58.95, 79.63, 126.83, 127.58, 128.88, 129.61, 135.28, 137.96, 172.79; IR (KBr) 3320, 3276, 1754, 1464 cm⁻¹. Anal. Calcd for C₁₁H₁₁NO₂: C, 69.83; H, 5.86; N, 7.40. Found: C, 69.72; H, 5.92; N, 7.24.
**(3*R*,4*S*)-3-Hydroxy-4-(2-methylpropyl)-2-azetidinone (3c):** 94%; white solid; mp 141-142°C; [α]D²⁰ +26.6° (c 0.70, MeOH); ¹H NMR (300 MHz, MeOH-d4) d 0.94 (d, J = 6.8 Hz, 3H), 0.97 (d, J = 6.8 Hz, 3H), 1.45 (m, 2H), 1.71 (sept, J = 6.6 Hz, 1H), 3.75 (m, 1H), 4.79 (d, J = 4.7 Hz, 1H); ¹³C NMR (75 MHz, MeOH-d4) δ 22.62, 23.48, 26.53, 39.90, 55.47, 77.76, 173.18; IR (KBr) 3274, 3178, 1762, 1685, 1155 cm⁻¹. Anal. Calcd for C₇H₁₃NO₂: C, 58.72; H, 9.15; N, 9.78. Found: C, 58.55; H, 9.41; N, 9.69.
**(3*R*,4*S*)-4-(Cyclohexylmethyl)-3-hydroxy-2-azetidinone (3d):** 92%; white solid; mp 147-148°C; [α]D²⁰ + 8.73° (c, 0.573, CH₃OH); ¹H NMR (300 MHz, MeOH-d4) δ 0.88-1.82 (m, 13H), 3.78 (m, 1H), 4.79 (d, J = 4.7 Hz, 1H); ¹H NMR (300 MHz, DMSO-d6) δ 0.86-1.72 (m, 13H), 3.58 (m, 1H), 4.63 (m, 1H), 5.82 (d, J = 7.6 Hz, 1H), 8.13 (d, J = 5.6, 1H); ¹³C NMR (75 MHz, MeOH-d4) δ 27.29, 27.41, 27.48, 34.07, 35.06, 36.11, 38.52, 55.02, 77.65, 173.22; IR (KBr) 3301, 3219, 2915, 2847, 1754, 1694, 1168 cm⁻¹. Anal.Calcd for C₁₀H₁₇NO₂: C, 65.54, H, 9.35, N, 7.64. Found: C, 65.72, H, 9.46, N, 7.42.
**(3*R*,4*S*)-4-cyclohexyl-3-hydroxy-2-azetidinone (3e):** A suspension of 500 mg (3.06 mmol) of 4-phenyl-3-hydroxy-2-azetidinone **1a** and 15 mg of Rh-C in 10 mL of methanol was heated at 90°C under 800 psi in an autoclave. After 5 days, the hydrogen pressure was released and the catalyst filtrated on celite. Evaporation of the solvent afforded a solid which was recrystallized in ethyl acetate to give 440 mg (85%) of **3e** as a white solid: White solid; mp 140-140.5°C; [α]_{D}²⁰ + 65.1° (c 0.66, CH₃OH); ¹H NMR (250 MHz, MeOH-d₄) δ 0.75-1.10 (m, 2H), 1.12-1.35 (m, 3H), 1.40-2.00 (m, 6H), 3.28 (dd, J = 9.7, 4.6 Hz, 1H), 4.81 (d, J = 4.6 Hz, 1H); ¹H NMR (250 MHz, DMSO-d₆) δ 0.75-1.00 (m, 2H), 1.10-1.35 (m, 3H), 1.37-1.55 (m, 1H), 1.58-1.85 (m, 5H), 3.10 (dd, J = 9.6, 4.7 Hz, 1H), 4.67 (m, 1H), 5.87 (d, J = 7.8 Hz, 1H), 8.21 (bs, 1H); ¹³C NMR (63 MHz, DMSO-d₆) δ 25.08, 25.36, 26.07, 28.83, 29.17, 37.51, 59.04, 76.41, 170.21; IR (KBr) 3312, 3219, 2928, 1726 cm⁻¹. Anal.Calcd for C₉H₁₅NO₂: C, 63.88, H, 8.93, N, 8.28. Found: C, 63.70, H, 9.00, N, 8.06.

### Examples 12-16

To a solution of 1.9 mmol of 3-hydroxy-4-substituted-2-azetidinone in 20 mL of THF, was added at 0°C 3.9 mmol of ethylvinylether. After 2 h, at 0°C, the reaction mixture was diluted with ether and washed with sat. NaHCO₃. The organic layer was dried over Na₂CO₃, filtered and concentrated to yield of 3-(1-ethoxyethoxy)-4-substituted-2-azetidinone:
**(3*R*,4*S*)-3-(1-Ethoxyethoxy)-4-phenyl-2-azetidinone (4a):** 100%; white solid; mp 78-80°C; ¹H NMR (CDCl₃) δ [0.98 (d, J = 5.4 Hz), 1.05 (d, J = 5.4 Hz), 3H], [1.11 (t, J = 7.1 Hz), 1.12 (t, J = 7.1 Hz), 3H], [3.16-3.26 (m), 3.31-3.42 (m), 3.59-3.69 (m), 2H], [4.47 (q, J=5.4 Hz), 4.68 (q, J = 5.4 Hz), 1H], [4.82 (d, J = 4.7 Hz), 4.85 (d, J = 4.7 Hz), 1H], 5.17-5.21 (m, 1H), 6.42 (bd, 1H), 7.35 (m, 5H); IR (KBr) 3214, 2983, 2933, 1753 , 1718 , 1456 cm⁻¹. Anal. Calcd for C₁₃H₁₇NO₃: C, 66.36; H, 7.28; N, 5.95. Found: C, 66.46; H, 7.11; N, 5.88.
**(3*R*,4*S*)-3-(1-Ethoxyethoxy)-4-(2-phenylethenyl)-2-azetidinone (4b):** 98%; white solid; mp 98-99°C; ¹H NMR (300 MHz, CDCl₃) δ [1.17 (t, J = 7.1 Hz), 1.18 (t, J = 7.1 Hz), 3H], [1.26 (d, J = 5.4 Hz), 1.35 (d, J = 5.4 Hz), 3H], [3.44-3.52 (m), 3.60-3.68 (m), 3.75-3.82 (m), 2H], 4.41 (dd, J = 4.9, 8.5 Hz, 1H), [4.81 (q, J = 5.4 Hz), 4.90 (q, J = 5.4 Hz), 1H], [5.11 (d, J = 4.9 Hz), 5.12 (d, J = 4.9 Hz), 1H], 6.01 (bs, 1H), [6.27 (dd, J = 8.5, 15.9 Hz), 6.28 (dd, J = 8.5, 15.9 Hz), 1H], [6.61 (d, J = 15.9 Hz), 6.63 (d, J = 15.9 Hz), 1H], 7.27-7.42 (m, 5H); ¹³C NMR (75 MHz, CDCl₃) δ 15.04, 20.37, 20.42, 57.22, 57.81, 61.23, 62.22, 78.77, 79.29, 99.50, 99.82, 125.56, 125.79, 126.59, 128.12, 128.65, 134.47, 134.58, 136.15, 168.59, 168.77; IR (KBr) 3310, 3030, 2963, 1770 cm⁻¹. Anal. Calcd for C₁₅H₁₉NO₃: C, 68.94; H, 7.33; N, 5.36. Found: C, 69.13; H, 7.44; N, 5.16.
**(3*R*,4*S*)-3-(1-Ethoxyethoxy)-4-(2-methylpropyl)-2-azetidinone (4c):** 100%; colorless oil: [α]D²⁰ +20.93° (c 1.72, CHCl₃); ¹H NMR (300 MHz, CDCl₃) δ 0.86 (d, J = 6.5 Hz, 3H), 0.92 (d, J = 6.5 Hz, 3H), 1.17 (t, J = 7.0 Hz, 3H), [1.29 (d, J = 5.3 Hz), 1.34 (d, J = 5.3 Hz), 3H], 1.46 (m, 2H), 1.62 (m, 1H), [3.49 (m), 3.69 (m), 2H)], 3.80 (m, 1H), [4.79 (q, J = 5.4 Hz), 4.90 (q, J = 5,4 Hz), 1H], 4.87 (m, 1H), 6.78 (bs, 1H); ¹³C NMR (75 MHz, CDCl₃) δ 15.08, 20.42, (21.98, 22.06), (23.15, 23.22), 25.35, (39.01, 39.10), (53.35, 53.69), (61.24, 62.24), (77.79, 77.92), (99.75, 100.05), (169.56, 169.65); IR (neat) 3269, 2956, 2871, 1758, 1468, 1382, 1340, 1152, 1115, 1083, 1052, 936, 893 cm⁻¹.
**(3*R*,4*S*)-4-(Cyclohexylmethyl)-3-(1-ethoxyethoxy)-2-azetidinone (4d):** 100%; colorless oil; [α]_{D}²⁰ + 10.92° (c 1.42, CHCl₃); ¹H NMR (300 MHz, CDCl₃) δ 0.84-1.71 (m, 13H), 1.16 (t, J = 7.0 Hz, 3H), [1.28 (d, J = 5.3 Hz), 1.33 (d, J = 5.3 Hz), 3H], 3.48 (m, 1H), [3.72 (m), 3.8 (m), 2H], [4.78 (q, J = 5.4 Hz), 4.85 (q, J=5.4 Hz), 1H], 4.82 (m, 1H), 6.76 (bs, 1H); ¹³C NMR (75 MHz, CDC₁₃) δ 14.37, 19.72, 25.30, 25.44, 25.63, (32.02, 32.13), (33.09, 33.17), (34.03, 34.07), (36.98, 37.07), (52.15, 52.49), (60.49, 61.52), (75.97, 76.39), (99.00, 99.35), (168.98, 169.05); IR (neat) 3278, 2924, 2852, 1758, 1448, 1382, 1150, 1114, 1086, 938, 886 cm⁻¹. Anal. Calcd for C₁₄H₂₅NO₃: C,65.85; H, 9.87; N, 5.49. Found: C, 66.03; H, 9.71; N, 5.30.
**(3*R*,4*S*)-4-Cyclohexyl-3-(1-ethoxyethoxy)-2-azetidinone (4e):** 100%; white solid; mp 87-89°C; [α]_{D}²⁰ + 83° (c 0.76, CH₃OH); ¹H NMR (250 MHz, CDCl₃) δ 0.84 (m, 2H), 1.07-1.34 (m, 9H), 1.66 (m, 6H), 3.32 (m, 1H), [3.42 (q, J = 7.7 Hz), 3.54 (q, J = 7.7 Hz), 3.65 (q, J = 7.7 Hz), 3.74 (q, J = 7.7 Hz), 2H], 4.81 (m, 1H), [4.80 (m), 4.90 (q, J = 5.2 Hz), 1H], 6.92 (bs, 1H); IR (CHCl₃) 3412, 2989, 2931, 1760, 1443, 1155, 1114 cm⁻¹. Anal. Calcd for C₁₃H₂₇NO₃: C, 64.70; H, 9.61; N, 5.80. Found: C, 64.82; H, 9.66; N, 5.64.

### Examples 17-32

To a solution of 2.2 mmol of 3-(1-ethoxyethoxy)-4-substituted-2-azetidinone, 5 mg of DMAP, 4.5 mmol of triethylamine in 20 mL of dichloromethane, was added dropwise at 0°C 3.3 mmol of alkylchloroformate dissolved in 5 mL of dichloromethane. The reaction mixture was stirred overnight at room temperature. The organic layer was washed several times with brine, dried over Na₂CO₃ and concentrated. The crude solid was purified by chromatography on silica gel to yield N-protected β-laetam:
**(3*R*,4*S*)-1-Methoxycarbonyl-3-(1-ethoxyethoxy)-4-phenyl-2-azetidinone (5a):** 62%; pale yellow oil; [α]D²⁰ +98.2° (c 1.1, CHCl₃); ¹H NMR (250 MHz, CDCl₃) δ [0.97 (d, J = 5.4 Hz), 1.08 (d, J = 5.4 Hz), 3H], 1.10 (bt, J = 7.3 Hz, 3H), [3.21 (dq, J = 9.5, 7.1 Hz), 3.32 (q, J = 7.1 Hz), 3.64 (dq, J = 9.5, 7.1 Hz), 2H], [3.76 (s), 3.77 (s), 3H], [4.48 (q, J = 5.4 Hz), 4.69 (q, J = 5.4 Hz), 1H], [5.11 (d, J = 5.9 Hz), 5.14 (d, J = 5.9 Hz), 1H], 5.23 (d, J = 5.9 Hz, 1H), 7.34 (m, 5H); ¹³C NMR (63 MHz, CDCl₃) δ (14.96, 15.07), (19.84, 20.69), 53.59, (60.74, 62.36), (61.14, 61.92), (76.21, 77.21), (99.16, 99.56), (127.73, 128.03, 128.31, 128.36, 128.62, 128.85), (133.41, 133.58), (149.51, 149.57), (165.21, 165.67); IR (neat) 3033, 2979, 2957, 1821, 1738, 1654, 1440, 1336, 1101 cm⁻¹. Anal. Calcd for C₁₅H₁₉NO₅: C, 61.42; H, 6.53; N, 4.78. Found: C, 61.55; H, 6.51; N, 4.90.
**(3*R*, 4*S*)-1-Ethoxycarbonyl-3-(1-ethoxyethoxy)-4-phenyl-2-azetidinone (5b):** 82%; colorless oil; [α]D²⁰ +100.9° (c 1.08, CHCl₃); ¹H NMR (250 MHz, CDCl₃) δ [0.95 (d, J = 5.4 Hz), 1.06 (d, J = 5.4 Hz), 3H], 1.08 (bt, J = 7.3 Hz, 3H), [1.19 (t, J = 7.1 Hz), 1.20 (t, J = 7.1 Hz), 3H], [3.20 (dq, J = 9.4, 7.1 Hz), 3.31 (q, J = 7.1 Hz), 3.32 (q, J = 7.1 Hz), 3.63 (dq, J = 9.4, 7.1 Hz), 2H], [4.18 (q, J = 7.1 Hz), 4.19 (q, J = 7.1 Hz), 2H], [4.47 (q, J = 5.4 Hz), 4.67 (q, J = 5.4 Hz), 1H], [5.09 (d, J = 5.8 Hz), 5.13 (d, J = 5.8 Hz), 1H], 5.21 (d, J = 5.8 Hz, 1H), 7.30 (m, 5H); ¹³C NMR (63 MHz, CDCl₃) δ 14.14, (14.95, 15.07), (19.86, 20.05), (60.76, 62.35), 62.36, (61.14, 61.90), (76.18, 77.20), (99.17, 99.53), (127.73, 128.02, 128.25, 128.30, 128.50, 128.63), (133.59, 133.77), (148.99, 149.05), (165.33, 165.79); IR (neat) 2978, 2934, 1814, 1731, 1646, 1540, 1456, 1323, 1175, 1096 cm⁻¹. Anal. Calcd for C₁₆H₂₁NO₅: C, 62.53; H, 6.89; N, 4.56. Found: C, 62.45; H, 6.63; N, 4.83.
**(3*R*,4S*)*-1-*n*-Butoxycarbonyl-3-(1-ethoxyethoxy)-4-phenyl-2-azetidinone (5c):** 83%; colorless oil; [α]D²⁰ +70.4° (c 1.25, CHCl₃); ¹H NMR (250 MHz, CDCl₃) δ 0.79 (t, J = 7.3 Hz, 3H), [0.94 (d, J = 5.1 Hz), 1.07 (d, J = 5.1 Hz), 3H], 1.07 (t, J = 7.4 Hz, 3H), 1.20 (m, 2H), 1.51 (quint, J = 6.7 Hz, 2H), [3.21 (m), 3.30 (q, J = 7.1 Hz), 3.61 (m), 2H], 4.09 (m, 2H), [4.46 (q, J = 5.2 Hz), 4.66 (q, J = 5.2 Hz), 1H], [5.07 (d, J = 5.8 Hz), 5.11 (d, J = 5.8 Hz), 1H], 5.19 (d, J = 5.8 Hz, 1H), 7.28 (m, 5H); ¹³C NMR (63 MHz, CDCl₃) δ 13.50, (14.95, 15.29), 18.71, (19.84, 20.05), 30.42, (60.77, 62.33), (61.25, 62.02), 66.51, (76.24, 77.26), (99.17, 99.52), (127.76, 128.03, 128.22, 128.27, 128.50, 128.60), (133.61, 133.80), (148.96, 149.02), (165.40, 165.85); IR (neat) 2961, 2933, 1817, 1732, 1653, 1456, 1394, 1250, 1099 cm⁻¹. Anal. Calcd for C₁₈H₂₅NO₅: C, 64.46; H, 7.51; N, 4.18. Found: C, 64.44; H, 7.57; N, 4.24.
**(3*R*,4*S*)-1-*tert*-Butoxycarbonyl-3-(1-ethoxyethoxy) -4-phenyl-2-azetidinone (5d):** 83%; white solid; mp 90-91°C; [α]D²⁰ +70.4° (c 1.25, CHCl₃); ¹H NMR (250 MHz, CDCl₃) δ [0.96 (d, J = 5.4 Hz), 1.08 (d, J = 5.4 Hz), 3H], [1.09 (t, J = 7.0 Hz), 1.10 (t, J = 7.0 Hz), 3H], [1.36 (s), 1.37 (s), 9H], [3.23 (dq, J = 9.5, 7.1 Hz), 3.32 (q, J = 7.1 Hz), 3.65 (dq, J = 9.5, 7.1 Hz), 2H], [4.48 (q, J = 5.4 Hz), 4.69 (q, J = 5.4 Hz), 1H], [5.03 (d, J = 5.8 Hz), 5.07 (d, J = 5.8 Hz), 1H], 5.18 (d, J = 5.8 Hz, 1H), 7.31 (m, 5H); ¹³C NMR (63 MHz, CDCl₃) δ (14.98, 15.08), (19.89, 20.10), 27.84, (60.74, 62.32), (61.28, 62.08), (75.91, 76.54), 83.48 (99.10, 99.41), (127.76, 128.07, 128.20, 128.42, 128.85), (133.98, 134.16), 147.56, (165.61, 166.04); IR (CHCl₃) 3025, 2982, 2932, 1809, 1725, 1601, 1497, 1331, 1256, 1152 cm⁻¹. Anal. Calcd for C₁₈H₂₅NO₅: C, 64.46; H, 7.51; N, 4.18. Found: C, 64.50; H, 7.41; N, 4.17.
**(3*R*,4*S*)-3-(1-Ethoxyethoxy)-1-phenoxycarbonyl -4-phenyl-2-azetidinone (5e):** 79%; white solid; mp 50-52°C; [α]D²⁰ +64.9° (c 0.94, CHCl₃) ; ¹H NMR (250 MHz, CDCl₃) δ [1.00 (d, J = 5.3 Hz), 1.11 (m), 3H], [1.14 (m), 3H], [3.27 (m), 3.35 (q, J = 7.1 Hz), 3.70 (m), 2H], [4.54 (q, J = 5.3 Hz), 4.74 (q, J = 5.3 Hz), 1H], [5.25 (d, J = 5.8 Hz), 5.29 (d, J = 5.8 Hz), 1H], 5.34 (d, J = 5.8 Hz, 1H), 7.03-7.39 (m, 10H); IR (CHCl₃) 3028, 2981, 2934, 1815, 1744, 1591, 1486, 1327, 1192 cm⁻¹. Anal. Calcd for C₂₀H₂₁NO₅: C, 67.59; H, 5.96; N, 3.94. Found: C, 67.33; H, 6.06; N, 3.75.
**(3*R*,4*S*)-3-(1-Ethoxyethoxy)-4-phenyl-1-phenyl methoxycarbonyl-2-azetidinone (5f):** 44%; white solid; mp 58-60°C; [α]D²⁰ +91.4° (c 1.16, CHCl₃); ¹H NMR (250 MHz, CDCl₃) δ [0.97 (d, J = 5.3 Hz), 1.09 (d, J = 5.3 Hz), 3H], [1.10 (t, J = 7.0 Hz), 1.11 (t, J = 7.0 Hz), 3H], [3.23 (dq, J = 9.5, 7.1 Hz), 3.33 (q, J = 7.1 Hz), 3.66 (dq, J = 9.5, 7.1 Hz), 2H], [4.50 (q, J = 5.4 Hz), 4.70 (q, J = 5.4 Hz), 1H], [5.13 (d, J = 5.6 Hz), 5.15 (d, J = 5.6 Hz), 1H], [5.19 (s), 5.20 (s), 2H], 5.23 (d, J = 5.6 Hz, 1H), 7.21 (m, 2H), 7.26-7.37 (m, 8H); ¹³C NMR (63 MHz, CDCl₃) δ (14.99, 15.10), (19.90, 20.10), (60.83, 62.41), (61.64, 62.14), 68.01, (76.31, 77.28), (99.19, 99.53), (127.37, 127.86, 128.07, 128.16, 128.36, 128.52, 128.63, 128.85), (133.49, 133.68), 134.89, (148.72, 148.78), (165.37, 165.81); IR (CHCl₃) 3028, 2981, 2934, 1815, 1733, 1604, 1450, 1380, 1004 cm⁻¹. Anal. Calcd for C₂₁H₂₃NO₅: C, 68.28; H, 6.28; N, 3.79. Found: C, 68.07; H, 6.43; N, 3.72.
**(3*R*,4*S*)-1-*tert*-Butoxycarbonyl-4-cyclohesyl-3-(1-ethoxyethoxy)-2-azetidinone (5g):** 91%; colorless oil; [α]_{D}²⁰ +62.5° (c 1.12, CHCl₃); ¹H NMR (250 MHz, CDCl₃) δ 1.10-1.28 (m, 6H), 1.15 (t, J = 7.0 Hz, 3H), [1.27 (d, J = 5.4 Hz), 1.31 (d, J = 5.4 Hz), 3H], [1.45 (s), 1.46 (s), 9H], 1.63-1.70 (m, 5H), [3.43 (dq, J = 9.2, 7.0 Hz), 3.62 (m), 3.75 (d, J = 7.0 Hz), 3.78 (d, J = 7.0 Hz), 2H], 3.85 (t, J = 6.1 Hz, 1H), [4.78 (q, J = 5.4 Hz), 4.88 (m), 1H], [4.85 (d, J = 6.1 Hz), 4.86 (d, J = 6.1 Hz), 1H]; ¹³C NMR (63 MHz, CDCl₃) δ 15.07, (20.25, 20.37), (26.05, 26.14), 26.26, (27.33, 27.95), (29.05, 29.20), (30.04, 30.23), (37.54, 37.64), (61.19, 62.53), (62.06, 62.32), (75.42, 75.85), 83.06, 100.11, 148.72, (166.70, 166.76); IR (neat) 2980, 2931, 2854, 1807, 1725, 1450, 1370, 1329, 1212, 1118 cm⁻¹. Anal. Calcd for C₁₈H₃₁NO₅: C, 63.32; H, 9.15; N, 4.10. Found: C, 63.15; H, 8.97; N, 3.96.
**(3*R*,4*S*)-1-*tert*-Butoxycarbonyl-3-(1-ethoxy ethoxy)-4-(2-phenylethenyl)-2-azetidinone (5h):** 86%; white solid; mp 69-73°C; ¹H NMR (300 MHz, CDCl₃) δ [1.16 (t, J = 7.1 Hz), 1.18 (t, J = 7.1 Hz), 3H], [1.25 (d, J = 5.4 Hz), 1.36 (d, J = 5.4 Hz), 3H], 1.48 (s, 9 H), [3.47 (m), 3.62 (m), 3.80 (m), 2H], 4.68 (dd, J = 5.8, 8.8 Hz, 1H), [4.82 (q, J = 5.4 Hz), 4.91 (q, 5.4 Hz), 1H], [5.09 (d, J = 5.8 Hz), 5.11 (d, J = 5.8 Hz), 1H], [6.23 (dd, J = 8.8, 15.8 Hz), 6.25 (dd, J = 8.8, 15.8 Hz), 1H], [6.72 (d, J = 15.8 Hz), 6.73 (d, J = 15.8 Hz), 1H], 7.27-7.44 (m, 5H); ¹³C NMR (75 MHz, CDCl₃) δ 14.98, 20.31, 27.98, 60.24, 60.85, 61.46, 62.36, 63.58, 83.38, 99.63, 99.87, 122.45, 122.63, 126.69, 128.20, 128.61, 136.15, 136.34, 136.38, 147.74, 147.79, 165.33, 165.53; IR (KBr) 3027, 3020, 2984, 2933, 1809, 1723 cm⁻¹. Anal. Calcd for C₂₀H₂₇NO₅: C, 66.46; H, 7.53; N, 3.88. Found: C, 66.60; H, 7.50; N, 3.87.
**(3*R*,4*S*)-1-*tert*-Butoxycarbonyl-3-(1-ethoxy ethoxy)-4-(2-methylpropyl)-2-azetidinone (5i):** 80%; yellow oil; [α]D²⁰ +77.45° (c 0.216, CHCl₃); ¹H NMR (300 MHz, CDCl₃) δ 0.89 (d, J = 5.7 Hz, 6H), 1.41 (t, J = 7.1 Hz, 3H), [1.25 (d, J = 5.3 Hz), 1.31 (d, J = 5.3 Hz), 3H], 1.45 (s, 9H), 1.51-1.67 (m, 3H), [3.48 (dq, J = 9.3, 7.1 Hz), 3.55-3.71 (m, 1H), 3.80 (dq, J = 9.3, 7.1 Hz), 2H], 4.08 (q, J = 6.1 Hz, 1H), [4.70 (q, J = 5.3 Hz), 4.90 (q, J = 5.3 Hz), 1H], 4.85 (d, J = 6.1 Hz, 1H); ¹³C NMR (75 MHz, CDCl₃) δ 14.95, (20.11, 20.28), (22.42, 22.59), 22.70, (24.89, 25.07), 27.83, (37.03, 37.31), (56.14, 56.38), (61.07, 62.27), (75.65, 75.92), 82.98, 99.91, 148.1, (166.1, 165.9); IR (neat) 2931, 2960, 2872, (1790, 1807), (1708, 1726), (1454, 1465), 1332, 1256, 1048, 1158, 996, 955, 857, 834, 770 cm⁻¹. Anal. Calcd for C₁₆H₂₆NO₅: C, 60.93; H, 9.27; N, 4.44. Found: C, 61.19; H, 9.41; N, 4.37.
**(3*R*,4*S*)-1-*tert*-Butoxycarbonyl-4-cyclohexyl methyl-3-(1-ethoxyethoxy)-2-azetidinone (5j):** 93%; yellow oil; [α]D²⁰ +75.64° (c 0.78, CHCl₃); ¹H NMR (300 MHz, CDCl₃) δ 0.81-1.74 (m, 13H), 1.19 (t, J = 7.1 Hz, 3H), 1.48 (s, 9H), [1.30 (d, J = 5.3 Hz), 1.35 (d, J = 5.3 Hz), 3H], [3.45 (dq, J = 9.3, 7.1 Hz), 3.62-3.71 (m), 3.78 (dq, J = 9.3, 7.1Hz), 2H], 4.01 (m, 1H), [4.81 (q, J = 5.3 Hz), 4.91 (q, J = 5.3 Hz), 1H], [4.86 (d, J = 6.1 Hz), 4.87 (d, J = 6.1 Hz), 1H]; ¹³C NMR (75 MHz, CDCl₃) δ 15.03, 20.19, 20.36, 26.10, 26.36, 27.91, (33.17, 33.31), (33.35, 33.49), (34.33, 34.58), (35.39, 35.68), (55.77, 55.99), (61.14, 62.21), (75.74, 75.90), 82.96, (99.86, 99.95), 147.96, 166.13; IR (neat) 2979, 2923, 2850, 1719, 1807, 1449, 1336, 1154 cm⁻¹. Anal. Calcd. for C₁₉H₃₃NO₅: C, 64.20; H, 9.36; N,3.94. Found: C, 64.00; H, 9.17; N, 4.02.

### Examples 28-32

To a solution of 0.5 mmol of 3-(1-ethoxyethoxy)-4-phenyl-2-azetidinone in 6 mL of tetrahydrofuran, was added dropwise at -78°C 0.6 mmol of n-BuLi. After 5 min, 1 mmol of an isocyanate or an isothiocyanate was added. The reaction mixture was stirred 30 min at -78°C and quenched by addition of 2 mL sat. NH₄Cl solution. The reaction mixture was diluted with 30 mL of ether and the organic layer was washed several times with brine, dried over Na₂CO₃ and concentrated. The crude solid was purified by chromatography on silica gel to yield N-protected β-lactam:
**(3*R*,4*S*)-3-(1-Ethoxyethoxy)-1-phenylcarbamoyl-4-phenyl-2-azetidinone (7a):** 66%; pale yellow solid; mp 152-155°C; [α]D²⁰ +87.8° (c 0.9, CHCl₃); ¹H NMR (250 MHz, CDCl₃) δ [1.07 (d, J = 5.4 Hz), 1.13 (d, J = 5.4 Hz), 3H], 1.16 (t, J = 7.1 Hz, 3H), [3.26 (dq, J = 9.5, 7.1 Hz), 3.37 (q, J = 7.1 Hz), 3.39 (q, J = 7.1Hz), 3.67 (dq, J = 9.5, 7.1 Hz), 2H], [4.53 (q, J = 5.4 Hz), 4.72 (q, J = 5.4 Hz), 1H], 5.28 (m, 2H), [6.59 (bs), 6.60 (bs), 1H], 7.10-7.55 (m, 10H), 8.68 (bs, 1H); ¹³C NMR (63 MHz, CDCl₃) δ (15.04, 15.16), (19.98, 20.11), (60.99, 62.53), 61.80, (76.05, 76.66), (99.34, 99.70), (119.63, 120.69, 124.37, 127.67, 127.95, 128.40, 128.45, 128.67, 128.85, 129.04, 129.12, 130.49), 133.48, (137.03, 137.28), (147.23, 147.29), (168.12, 168.52); IR (CHCl₃) 3342, 3017, 2982, 2932, 1773, 1719, 1602, 1548, 1445, 1312, 1224, 1210 cm⁻¹. Anal. Calcd for C₂₀H₂₂N₂O₄: C, 67.78; H, 6.26; N, 7.90. Found: C, 67.92; H, 5.98; N, 8.17.
**(3*R*,4*S*)-1-*tert*-Butylcarbamoyl-3-(1-ethoxy ethoxy)-4-phenyl-2-azetidinone (7b):** 74%; pale yellow viscous oil; [α]D²⁰ +144.3° (c 0.7, CHCl₃); ¹H NMR (250 MHz, CDCl₃) δ [0.96 (d, J = 5.3 Hz), 1.05 (d, J = 5.3 Hz), 3H], 1.10 (t, J = 7.1 Hz, 3H), [1.33 (s), 1.34 (s), 9H], [3.21 (dq, J = 9.3, 7.0 Hz), 3.20 (q, J = 7.0 Hz), 3.33 (q, J = 7.1Hz), 3.62 (dq, J = 9.1, 7.0 Hz), 2H], [4.46 (q, J = 5.4 Hz), 4.66 (q, J = 5.4 Hz), 1H], 5.10-5.19 (m, 2H), [6.59 (bs), 6.60 (bs), 1H], 7.23-7.36 (m, 5H); ¹³C NMR (63 MHz, CDCl₃) δ (14.86, 14.99), (19.75, 19.95), (28.81, 29.30), (60.62, 61.20), (60.80, 62.29), (75.57, 76.76), (98.91, 99.34), (127.07, 127.40, 127.70, 128.17, 128.29, 128.53), (133.71, 133.86), (148.54, 148.59), (167.67, 168.13); IR (CHCl₃) 3362, 3035, 2977, 2932, 1767, 1710, 1605, 1537, 1457, 1366, 1320, 1282, 1217, 1100 cm⁻¹. Anal. Calcd for C₁₈H₂₆N₂O₄: C, 64.65; H, 7.84; N, 8.38. Found: C, 64.46; H, 7.75; N, 8.39.
**(3*R*,4*S*)-1-Benzylcarbamoyl-3-(1-ethoxy ethoxy)-4-phenyl-2-azetidinone (7c):** 50%; pale yellow viscous oil; [α]D²⁰ +66.2° (c 0.8, CHCl₃); ¹H NMR (250 MHz, CDCl₃) δ [0.99 (d, J = 5.5 Hz), 1.08 (d, J = 5.5 Hz), 3H], 1.12 (m, 3H), [3.16-3.40 (m), 3.63 (m), 2H], [4.35-4.55 (m), 4.69 (q, J = 5.5 Hz), 3H], 5.21 (m, 2H), [7.03 (bs), 7.05 (bs), 1H], 7.32 (m, 10H); ¹³C NMR (63 MHz, CDCl₃) δ (15.01, 15.14), (19.90, 20.11), 43.83, (60.66, 62.44), (60.75, 61.54), (75.93, 77.04), (99.16, 99.56), (127.25, 127.64, 127.69, 128.17, 127.93, 128.35, 128.55, 128.64, 128.74), (133.59, 133.76), 137.80, 150.02, (167.73, 168.19); IR (CHCl₃) 3379, 3090, 3033, 2980, 2930, 1773, 1707, 1604, 1536, 1455, 1319, 1270, 908 cm⁻¹. Anal. Calcd for C₂₁H₂₄N₂O₄: C, 68.46; H, 6.57; N, 7.60. Found: C, 68.30; H, 6.66; N, 7.51.
**(3*R*,4*S*)-3-(1-Ethoxyethoxy)-1-ethylcarbamoyl-4-phenyl-2-azetidinone (7d):** 63%; pale yellow oil; [α]D²⁰ +96.7° (c 0.9, CHCl₃); ¹H NMR (250 MHz, CDCl₃) d [0.96 (d, J = 5.3 Hz), 1.04 (d, J = 5.3 Hz), 3H], 1.05-1.18 (m, 3H), [3.13-3.39 (m), 3.59 (m), 4H], [4.45 (q, J = 5.3 Hz), 4.65 (q, J = 5.3 Hz), 1H], 5.16 (m, 2H), [6.60 (bs), 6.62 (bs), 1H], 7.27 (m, 5H); ¹³C NMR (63 MHz, CDCl₃) δ 14.98, (19.84, 29.93), 34.79, (60.56, 61.35), (60.72, 62.35), (75.91, 77.03), (99.14, 99.54), (127.28, 127.55, 127.85, 128.27, 128.40), (133.74, 133.89), (149.87, 149.93), (167.62, 168.07); IR (CHCl₃) 3378, 3035, 2980, 2934, 1774, 1704, 1537, 1455, 1321, 1271, 1112, 1025 cm⁻¹.
**(3*R*,4*S*)-3-(1-Ethoxyethoxy)-1-phenylthio carbamoyl-4-phenyl-2-azetidinone (7e):** 82%; yellow solid; mp 108-112°C; [α]D²⁰ +68° (c 1.14, CHCl₃); ¹H NMR (250 MHz, CDCl₃) δ [1.02 (d, J = 5.5 Hz), 1.11 (d, J = 5.5 Hz), 3H], 1.16 (t, J = 7.3 Hz, 3H), [3.20-3.44 (m), 3.66 (dq, J = 9.4, 7.3 Hz), 2H], [4.52 (q, J = 5.5 Hz), 4.72 (q, J = 5.5 Hz), 1H], [5.30 (d, J = 5.5 Hz), 5.32 (d, J = 5.5 Hz), 1H], [5.49 (d, J = 5.5 Hz), 5.52 (d, J = 5.5 Hz), 1H], 7.36 (m, 8H), 7.67 (d, J = 7.8 Hz, 2H), 10.37 (bs, 1H); ¹³C NMR (63 MHz, CDCl₃) δ (15.04, 15.17), (19.95, 20.13), (60.96, 62.57), (63.92, 64.75), (74.75, 75.84), (99.34, 99.68), (123.43, 126.58, 127.91, 128.28, 128.49, 128.86, 128.91), (133.10, 133.25), (137.36), (166.55, 166.52), (174.812); IR (CHCl₃) 3288, 3024, 2983, 1760, 1497, 1385, 1222 cm⁻¹.

### Examples 33-34

**(3*R*,4*S*) -1-Morpholinecarbonyl-3-(-1-ethoxyethoxy)-4-phenyl-2-azetidinone (7f):** To a solution of 30 mg (0.13 mmol) of 3-(1-ethoxyethoxy)-4-phenyl-2-azedinone 6 in 2 mL of CH₂Cl₂, 2 mg of DMAP and 0.05 mL of triethylamine was added at room temperature. After 5 min. 22.9 mg (0.15 mmol) of morpholinecarbonyl chloride was added. The reaction mixture was stirred for 2h at room temperature. The reaction mixture was diluted with 20 mL of CH₂Cl₂ and the organic layer was washed two times with brine, dried over Na₂CO₃ and concentrated. The crude solid product was purified by chromatography on silica gel to yield pure 7f: 87%; pale yellow oil; ¹H NMR (250 MHz, CDCl₃) δ [0.90 (d, J = 5.3 Hz), 1.01 (d, J = 5.3 Hz) (3H)], [1.04 (t, J = 7.1 Hz), 1.18 (t, J = 7.1 Hz)] (3H), 3.20 (m, 4H), [3.28 (m), 3.53 (m), 3.67 (m), (2H)], 3.60 (m, 4H), [4.41 (g, J = 5.3 Hz), 4.63 (q, J = 5.3 Hz) (1H), [5.07 (d, J = 5.8 Hz), 5.08 (d, J = 5.8 Hz) (1H), [5.29 (d, J = 5.8 Hz), 5.32 (d, J = 5.8 Hz) (1H)], 7.23-7.27 (m, 5H).

### Examples 35-53

To a solution of 0.37 mmol of O-EE β-lactam in 4 mL THF was added 4 mL of 0.5 N HCl. The completion of reaction was monitored by TLC. After 1-3 hr, the reaction mixture was concentrated in vacuo to remove THF. The residue was dissolved in 30 mL ether and washed with 10 mL saturated NaHCO₃ solution. The ether layer was washed with brine, dried over anhydrous sodium sulfate, and concentrated in vacuo to give 3-hydroxy β-lactam:
**(3*R*,4*S*)-3-Hydroxy-1-methoxycarbonyl-4-phenyl-2-azetidinone (6a)**: 66%; white solid; mp ; 91-92°C [α]D²⁰ +108° (c 0.63, CHCl₃); ¹H NMR (250 MHz, CDCl₃) δ 3.80 (s, 3H), 5.13 (d, J = 6.0 Hz, 1H), 5.22 (d, J = 6.0 Hz, 1H), 7.25-7.42 (m, 5H); ¹³C NMR (63 MHz, CDCl₃) δ 53.77, 61.44, 77.33, 127.16, 128.94, 132.65, 149.20, 166.04; IR (CHCl₃) 3432, 3024, 2996, 1806, 1730, 1440, 1333, 1188 cm⁻¹. MS(FAB) m/z (%) 222 (M+1, 38), 194(29), 164(100).
**(3*R*,4*S*)-1-Ethoxycarbonyl-3-hydroxy-4-phenyl-2-azetidinone (6b):** 59%; white solid; mp 112-113°C; [α]_{D}²⁰ +181° (c 0.97, CHCl3) ; ¹H NMR (250 MHz, CDCl₃) δ 1.27 (t, J = 7.1 Hz, 3H), 4.25 (q, J = 7.1 Hz, 2H), 5.14 (d, J = 6.0 Hz, 1H), 5.22 (d, J = 6.0 Hz, 1H), 7.27-7.39 (m, 5H); ¹³C NMR (63 MHz, CDCl₃) δ 14.08, 61.36, 63.00, 77.26, 127.08, 128.83, 132.75, 149.08, 165.79; IR (CHCl₃) 3605, 3017, 2985, 1815, 1732, 1684, 1396, 1373, 1268, 1020 cm⁻¹; MS (FAB; m/z (%) 236 (M+1, 98), 208(23), 178(100).
**(3*R*,4*S*)-1-*n*-Butoxycarbonyl-3-hydroxy-4-phenyl-2-azetidinone (6c):** 69%; white solid; mp 88-89°C; [α]D²⁰ +159.1° (c 0.71, CHCl₃); ¹H NMR (250 MHz, CDCl₃) δ 0.78 (t, J = 7.3 Hz, 3H), 1.14 (m, 2H), 1.50 (m, 2H), [4.07 (q, J = 8.9 Hz), 4.10 (q, J = 8.9 Hz), 2H), 5.05 (d, J = 5.9 Hz, 1H), 5.11 (d, J = 5.9 Hz, 1H), 7.22-7.36 (m, 5H); ¹³C NMR (63 MHz, CDCl₃) δ 13.44, 18.71, 30.44, 61.54, 66.72, 77.31, 127.21, 128.80, 132.89, 149.15, 166.06; IR (CHCl₃) 3562, 3018, 2962, 1813, 1730, 1456, 1395, 1324, 1222, 1099 cm⁻¹. MS (FAB) m/z (%) 264(M+1,62), 236(20), 208(40), 206(100).
**(3*R*,4*S*)-1-*tert*-Butoxycarbonyl-3-hydroxy-4-phenyl-2-azetidinone (6d):** 88%; white solid; mp 131.5-132°C; [α]D²⁰ +173.5° (c 0.98, CHCl₃); ¹H NMR (250 MHz, CDCl₃) δ 1.40 (s, 9H), 2.70 (bs, 1H), 5.08 (d, J = 5.9 Hz, 1H), 5.14 (d, J = 5.9 Hz, 1H), 7.27 (d, J = 6.1 Hz, 2H), 7.38 (m, 3H); ¹³C NMR (63 MHz, CDCl₃) δ 27.87, 61.56, 77.00, 83.85, 127.20, 128.77, 128.82, 133.13, 147.72, 169.49; IR (CHCl₃) 3616, 3019, 2976, 1807, 1726, 1601, 1522, 1422, 1333, 1212, 1152 cm⁻¹. Anal. Calcd for C₁₄H₁₇NO₄: C, 63.87; H, 6.51; N, 5.32. Found: C, 63.71; H, 6.38; N, 5.12.
**(3*R*,4*S*)-3-Hydroxy-1-phenoxycarbonyl-4-phenyl-2-azetidinone (6e):** 72%; white solid; mp 125-126°C; [α]D²⁰ +107° (c 1.45, CHCl₃); ¹H NMR (250 MHz, CDCl₃) δ 5.21 (d, J = 6.1 Hz, 1H), 5.34 (d, J = 6.1 Hz, 1H), 7.07-7.45 (m, 10H); ¹³C NMR (63 MHz, CDCl₃) δ 61.83, 73.24, 121.15, 125.46, 126.80, 127.22, 128.09, 128.80, 129.11, 129.30, 132.40, 138.49, 154.05; IR (CHCl₃) 3615, 3020, 2976, 1821, 1740, 1506, 1487, 1332, 1219 cm⁻¹.
**(3*R*,4*S*)-1-Benzyloxycarbonyl-3-hydroxy-4-phenyl-2-azetidinone (6f):** 85%; white solid; mp 105-106°C; [α]D²⁰ +177° (c 0.6, CHCl₃); ¹H NMR (250 MHz, CDCl₃) δ 5.12 (d, J = 6.2 Hz, 1H), 5.22 (m, 3H), 7.24-7.40 (m, 10H); ¹³C NMR (63 MHz, CDCl₃) δ 61.53, 68.30, 77.43, 127.19, 128.13, 128.58, 129.06, 132.55, 134.74, 148.90, 165.92; IR (CHCl₃) 3557, 3018, 2924, 1814, 1731, 1383, 1273, 1162, 1004 cm⁻¹. MS (FAB) m/z (%) 298(M+1,14), 273(4).
**(3*R*,4*S*)-1-tert-Butorycarbonyl-4-cyclohexyl-3-hydroxy-2-azetidinone (6g):** 96%; white solid; mp 121-122°C; [α]D²⁰+78° (c 0.68, CHCl₃); ¹H NMR (250 MHz, CDCl₃) δ 1.17-1.75 (m, 11H), 1.48 (s, 9H), 3.83 (t, J+6.5 Hz, 1H), 4.96 (d, J=6.5 Hz, 1H); ¹³C NMR (63 MHz, CDCl₃) δ 25.87, 25.99, 26.24, 27.96, 29.69, 29.90, 37.45, 63.30, 75.24, 83.43, 148.80, 168.60; IR (CHCl₃) 3354, 2931, 2848, 1801, 1724, 1324, 1154 cm⁻¹.
**(3*R*,4*S*)-1-*tert*-Butoxycarbonyl-3-hydroxy-4-(2-phenylethenyl)-2-azetidinone (6h):** 96%; white solid; mp 132-133°C; [α]D²⁰ +122.0° (c 1.1, CHCl₃); ¹H NMR (300 MHz, CDCl₃) δ 1.47 (s, 9H), 3.88 (bs, 1H), 4.71 (dd, J = 4.8, 8.0 Hz, 1H), 5.07 (d, J = 4.8 Hz, 1H), 6.26 (dd, J = 8.0, 15.9 Hz, 1H), 6.72 (d, J = 15.9 Hz, 1H), 7.24-7.43 (m, 5H); ¹³C NMR (75 MHz, CDCl₃) δ 27.94, 60.78, 76.58, 83.77, 121.41, 126.75, 128.26, 128.59, 135.94, 136.62, 147.85, 166.95; IR (KBr) 3242, 3039, 2954, 1812, 1726 cm⁻¹. Anal. Calcd for C₁₆H₁₉NO₄: C, 66.42; H, 6.62; N, 4.84. Found: C, 66.31; H, 6.71; N, 4.76.
**(3*R*,4*S*)-1-*tert*-Butoxycarbonyl-3-hydroxy-4-(2-methylpropyl)-2-azetidinone (6i):** 98%; pale yellow solid; mp 108°C; [α]D²⁰ +76.14° (c 0.88, CHCl₃); ¹H NMR (300 MHz, CDCl₃) δ 0.93 (d, J = 6.3 Hz, 6H), 1.48 (s, 9H), 1.62-1.82 (m, 3H), 4.12 (m, 1H), 4 30 (bs, 1H), 4.93 (d, J = 5.9 Hz, 1H); ¹³C NMR (75 MHz, CDCl₃) δ 22.45, 22.78, 25.12, 27.96, 36.28, 57.59, 75.39, 83.46, 148.13, 168.00; IR (KBr) 3363, 2960, 2926, 1733, 1763, 1458, 1370, 1350, 1303, 1153 cm⁻¹. Anal. Calcd. for C₁₂H₂₁NO₄: C, 59.24; H, 8.70; N, 5.76. Found: C, 59.47; H, 8.91; N, 5.51.
**(3*R*,4*S*)-1-*tert*-Butoxycarbonyl-4-cyclohexyl methyl-3-hydroxy-2-azetidinone** (6j): 100%; white solid; mp 105-106°C; [α]D²⁰ +61.89° (c 0.74, CHCl₃); ¹H NMR (300 MHz, CDCl₃) δ 0.82-1.84 (m, 13H), 1.50 (s, 9H), 3.82 (bs, 1H), 4.14 (m, 1H), 4.93 (d, J = 5.8 Hz, 1H); ¹³C NMR (75 MHz, CDCl₃) δ 26.12, 26.17, 26.42, 33.20, 33.47, 33.59, 34.71, 28.00, 57.13, 75.49, 83.47. 148.08, 167.57; IR (KBr) 3442, 2921, 2850, 1797, 1682, 1447, 1354, 1342, 1159 cm⁻¹. Anal. Calcd. for C₁₅H₂₅NO₄: C, 63.58; H, 8.89; N, 4.94. Found: C, 63.76; H, 8.72; N, 4.68.
**(3*R*,4*S*)-3-hydroxy-4-phenyl-1-phenylcarbamoyl-2-azetidinone (8a):** 88%; white solid; mp 197-200°C; [α]D²⁰ +206.4° (c 1.26, CHCl₃); ¹H NMR (250 MHz, CD₃COCD₃) δ 5.39-5.47 (m, 2H), 7.07-7.60 (m, 10H), 8.80 (bs, 1H); ¹³C NMR (63 MHz, CD₃COCD₃) δ 61.98, 78.06, 119.85, 124.31, 128.11, 128.31, 128.60, 129.48, 135.31, 138.43, 148.17, 169.76; IR (CHCl₃) 3343, 3018, 2975, 1772, 1712, 1603, 1548, 1447, 1362, 1219, 1045 cm⁻¹; MS (FAB) m/z(%) 283(2), 263 (33) 207(22), 143(100).
**(3*R*,4*S*)-1-*tert*-Butylcarbamoyl-3-hydroxy-4-phenyl-2-azetidinone (8b):** 89%; white solid; mp 148-151°C; [α]D²⁰ +160.9° (c 1.28, CHCl₃); ¹H NMR (250 MHz, CDCl₃) δ 1.35 (s, 9H), 3.16 (bs, 1H), 4.97 (d, J = 5.5 Hz, 1H), 5.11 (d, J = 5.5 Hz, 1H), 6.60 (bs, 1H), 7.19-7.38 (m, 5H); ¹³C NMR (63 MHz, CDCl₃) δ 28.84, 51.53, 60.74, 76.61, 127.00, 128.61, 128.70, 133.13, 143.78, 168.30; IR (CHCl₃) 3362, 3018, 2975, 1767, 1710, 1533, 1422, 1318, 1216, 1045 cm⁻¹ Anal. Calcd for C₁₄H₁₈N₂O₃: C, 64.11; H, 6.92; N, 10.68. Found: C, 64.10; H, 7.08; N, 10.49.
**(3*R*,4*S*)-1-Benzylcarbamoyl-3-hydroxy-4-phenyl-2-azetidinone (8c)**: 63%; white solid; mp 165-168°C; [α]D²⁰ +139° (c 0.64, CHCl₃); ¹H NMR (300 MHz, CDCl₃) δ 3.10 (bs, 1H), 4.43 (dd, J = 15.2, 5.8 Hz, 1H), 4.50 (dd, J = 15.2, 5.8 Hz, 1H), 5.03 (d, J = 5.6 Hz, 1H), 5.20 (d, J = 5.6 Hz, 1H), 7.06 (t, J = 5.8 Hz, 1H), 7.23-7.33 (m, 10H); ¹³C NMR (63 MHz, CDCl₃) δ 43.79, 61.01, 76.94, 127.13, 127.73, 128.80, 128.86, 132.94, 137.59, 150.15, 168.34; IR (CHCl₃) 3364, 3028, 2925, 1771, 1704, 1537, 1455, 1361, 1219, 1190, 987 cm⁻¹. Anal. Calcd for C₇H₁₆N₂O₃: C₁ 68.91; H, 5.44; N. 9.45. Found: C₁ 68.89; H. 5.66; N, 9.34.
**(3*R*,4*S*)-1-Ethylcarbamoyl-3-hydroxy-4-phenyl-2-azetidinone (8d)**: 55%; white solid; mp 141- 42°C; [α]D²⁰ +211.4° (c 0.44, CHCl₃); ¹H NMR (250 MHz, CDCl₃) δ 1.19 (t, J = 7.2 Hz, 3H), 3.34 (qd, J = 7.2, 1.6 Hz, 2H), 5.09 (d, J = 5.6 Hz, 1H), 5.27 (d, J = 5.6 Hz, 1H), 6.63 (bt, J = 1.6 Hz, 1H), 7.23-7.44 (m, 5H); ¹³C NMR (63 MHz, CDCl₃) d 15.04, 34.94, 60.77, 76.98, 127.00, 128.92, 129.06, 132.83, 149.96, 167.98; IR (CHCl₃) 3381, 3018, 2990, 1770, 1732, 1651, 1589, 1422, 1298, 1210, 1045 cm⁻¹.
**(3*R*,4*S*)-3-(1-Hydroxy)-1-phenylthiocarbamoyl-4-phenyl-2-azetidinone (8e):** 78%; yellow solid; mp 85-88°C; [α]D²⁰ + 156.7° (c 0.67, CHCl₃); ¹H NMR (300 MHz, CDCl₃) δ 5.16 (d, J = 5.8 Hz, 1H), 5.53 (d, J = 5.8 Hz, 1H), 7.31-7.44 (m, 8H), 7.66 (d, J = 7.8 Hz, 2H), 10.33 (bs, 1H); ¹³C NMR (63 MHz, CDCl₃) δ 63.97, 75.72, 123.29, 126.49, 127.27, 128.77, 132.49, 137.26, 174.87; IR (CHCl₃) 3553, 3295, 3048, 2949, 1760, 1601, 1384, 1313 cm⁻¹; MS (FAB) m/z (%) 299(M+1, 46), 179(100).
**(3*R*,4*S*)-1-(Morpholinecarbonyl)-3-hydroxy-4-phenyl-2-azetidinone (8f):** 83%; white solid; mp 55-57°C; ¹H NMR (250 MHz, CDCl₃) δ 3.05 (bs, 1H), 3.56-3.78 (m, 8H), 5.00 (d, J = 5.9 Hz, 1H), 5.38 (d, J = 5.9 Hz, 1H), 7.24-7.40 (m, 5H).
**(3*R*,4*S*)-1-(N,N-Dimethylcarbamoyl)-3-hydroxy-4-phenyl-2-azetidinone (8g):** 88%; white crystal; mp 123-125°C; ₁H NMR (250 MHz, CDCl₃) δ3.06 (bs, 6H, 4.98 (d, J=5.9 Hz, 1H), 5.35 (d, J=5.9 Hz, 1H), 7.29-7.39 (m, 5H).
**(3*R*,4*S*)-1-*tert*-Butoxycarbonyl-4-phenyl-3-(1,1,1-trichloroethoxycarbonyl)-2-azetidinone (9a):** To a solution of 99 mg (0.38 mmol) of 1-*tert*-butylcarbonyl-3-hydroxy-4-phenyl- 2-azetidinone, 5 mg of DMAP and 263 mL (2 mmol) of triethylamine in 5 mL of dichloromethane, was added at 0°C 105 mL (0.8 mmol) of 1,1,1-trichloroethyl-chloroformate. The reaction mixture was stirred overnight at room temperature. The organic layer was washed several times with brine, dried over MgSO₄ and concentrated. The crude solid was purified by chromatography on silica gel to yield 65 mg (40%) of O-protected β-lactam: White solid; mp 122-124°C; [α]D²⁰ +28° (c 0.5, CHCl₃); ¹H NMR (250 MHz, CDCl₃) δ 1.39 (s, 9H), 4.43 (d, J = 11.7 Hz, 1H), 4.55 (d, J = 11.7 Hz, 1H), 5.28 (d, J = 5.5 Hz, 1H), 5.76 (d, J = 5.5 Hz, 1H), 7.30 (m, 5H); ¹³C NMR (63 MHz, CDCl₃) δ 27.81, 60.80, 77.03, 78.76, 84.40, 127.73, 128.58, 129.09, 131.55, 147.71, 152.17, 160.34; IR (CHCl₃) 3016, 2976, 1819, 1771, 1732, 1683, 1244 cm⁻¹. Anal. Calcd for C₁₇H₁₈Cl₃NO₆: C, 46.54; H, 4.14; N, 3.19. Found: C, 46.33; H, 4.34; N, 3.33.
**(3*R*,4*S*)-3-Acetoxy-1-*tert*-butoxycarbonyl-4-phenyl -2-azetidinone (9b):** To a solution of 82 mg (0.3 mmol) of 1-*tert*-butylcarbonyl-3-hydroxy-4-phenyl-2-azetidinone, 5 mg of DMAP and 210 mL (1.5 mmol) of triethylamine in 5 mL of dichloromethane, was added at 0°C 58 mL (0.7 mmol) of acetic anhydride. The reaction mixture was stirred overnight at room temperature. The organic layer was washed several times with brine, dried over MgSO₄ and concentrated. The crude solid was purified by chromatography on silica gel to yield 71 mg (75%) of O-acetyl β-lactam: White solid; mp 63-64°C; [α]D²⁰ +32.1° (c 0.81, CHCl₃); ¹H NMR (250 MHz, CDCl₃) δ 1.37 (s, 9H), 1.65 (s, 3H), 5.22 (d, J = 5.5 Hz, 1H), 5.83 (d, J = 5.5 Hz, 1H), 7.23-7.33 (m, 5H); ¹³C NMR (63 MHz, CDCl₃) δ 19.71, 27.81, 60.84, 75.94, 84.07, 127.43, 128.31, 128.67, 132.44, 147.25, 162.39, 168.83; IR (CHCl₃) 3026, 2984, 1815, 1752, 1731, 1497, 1371, 1286, 1224, 1152, 1024 cm⁻¹. Anal. Calcd for C₁₆H₁₉NO₅: C, 62.94; H, 6.27; N, 4.59. Found: C, 63.17; H, 6.14; N, 4.52.

### Example 5

To a solution of 90 mg (0.1 mmol) of 7, 10-ditroc-10-deacetylbaccatin III and 47 mg (0.14 mmol) of **5d** in 5 mL of THF, was added at -30°C 110 mL (0.11 mmol, 1M in THF) of sodium hexamethyldisilazide. The reaction was monitored by TLC and quenched by addition of brine. The aqueous layer was extracted with dichloromethane. The combined organic layers were washed with brine, dried over Na₂CO₃ and concentrated. The crude oil was purified by chromatography on silica gel using AcOEt/hexanes (1/2) as the eluant to give 117 mg of the coupling product 2'-EE-7, 10-ditroc-TAXOTÈRE as a white solid (94%).

The Troc protecting group was removed by stirring at 60°C 50 mg of 7,10-ditroc-TAXOTÈRE in 1mL ofMeOH and 1 mL of AcOH in presence of 150 mg of zinc for 1 hr. The reaction mixture was filtrated and diluted with dichloromethane. The organic phase was washed with sat. NaHCO₃ sol., brine dried over MgSO₄ and concentrated. The crude oil was purified by chromatography on silica gel using AcOEt/hexanes (1/1) as the eluant to give 28 mg (80%) of TAXOTÈRE as a white solid: [α]_{D}²⁰ -34°(c 0.7, EtOH); NMR (250 MHz, CDCl₃) δ 1.13 (s, 3H), 1.26 (s, 3H), 1.35 (s, 9H), 1.80 (s, 3H), 1.85 (m,), 1.90 (s, 3H), 2.24 (m, 2H), 2.39 (s, 3H), 2.55 (m,), 2.62 (m,), 3.53 (s,), 3.92 (d, J = 7.0 Hz,), 4.18 (d, J = 8.4 Hz), 4.22 (m,), 4.32 (d, J = 8.4 Hz,), 4.66 (d, J = 6.9 Hz,), 6.19 (bt, J = 8.1 Hz), 7.32-7.35 (m, 5H), 7.50 (t, J = 7.5 Hz, 2H), 7.62 (t, J = 7.3 Hz), 8.10 (d, J = 7.5 Hz, 2H). These data are consistent with those reported for TAXOTÈRE by Mangatal, L. et al. (Ref. Mangatal, L.; Adeline, M.T.; Guénard, D.; Guéritte-Voegelein, F.; Potier, P. *Tetrahedron* **1989**, 45, 4177.)

## Claims (Claims for the following Contracting State(s): PT)

1. A process for the preparation of a taxane derivative of the formula: 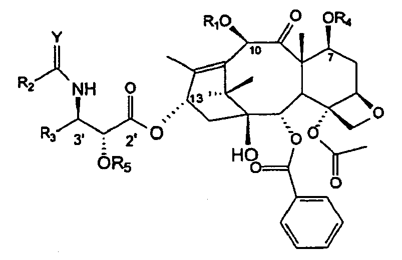 in which
R₁ represents a hydrogen or an acyl or an alkyl or an alkenyl or an alkynyl or a carbocyclic aryl or a heteroaryl radical or a hydroxyl protecting group;
R₂ represents an RO-, RS- or RR'N- in which R represents a straight chain or branched alkyl, alkenyl or alkynyl, cycloalkyl, heterocycloalkyl, cycloalkenyl, heterocycloalkenyl, carbocyclic aryl or heteroaryl radical, these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals, the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms; R' is hydrogen or R defined above; R and R' can be connected to form a cyclic structure;
Y is oxygen or sulfur;
R₃ represents a straight chain or branched alkyl, alkenyl or alkynyl, cycloalkyl, cycloalkenyl or carbocyclic aryl radical, these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl radicals, the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms;
R₄ represents hydrogen or an acyl radical or an unsubstituted straight chain or branched alkyl, alkenyl or alkynyl radical, an unsubstituted cycloalkyl, heterocycloalkyl, cycloalkenyl or heterocycloalkenyl radical, an unsubstituted aryl or heteroaryl radical, or a hydroxyl group protecting group;
R₅ represents hydrogen or an acyl radical or an unsubstituted straight chain or branched alkyl, alkenyl, or alkynyl radical, an unsubstituted cycloalkyl, heterocycloalkyl, cycloalkenyl or heterocycloalkenyl radical, an unsubstituted aryl or heteroaryl radical, or a hydroxyl protecting group;
which process comprises preparing a baccatin III derivative of the formula: 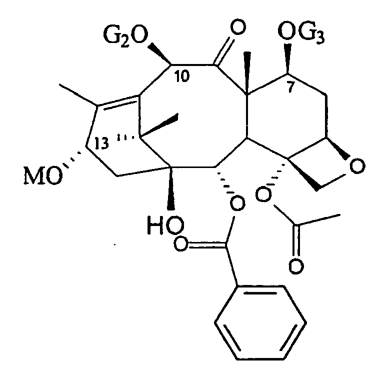 in which M is an alkali metal or alkaline earth metal atom (ion);
G₂ represents a hydroxyl protecting group or an acyl radical or an unsubstituted straight chain or branched alkyl, alkenyl, or alkynyl radical, an unsubstituted cycloalkyl, heterocycloalkyl, cycloalkenyl or heterocycloalkenyl radical, or an unsubstituted aryl or heteroaryl radical; and
G₃ represents a hydroxyl protecting group or an acyl radical or an unsubstituted straight chain or branched alkyl, alkenyl or alkynyl radical, an unsubstituted cycloalkyl, heterocycloalkyl, cycloalkenyl, or heterocycloalkenyl radical, or an unsubstituted aryl or heteroaryl radical,
by reacting a protected baccatin III of formula: 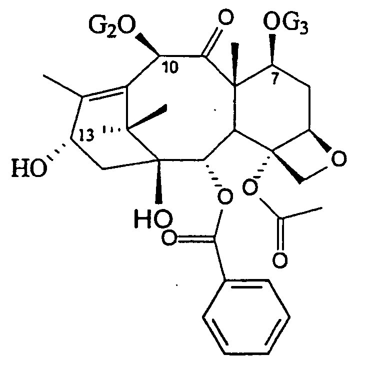 in which G₂ and G₃ are as defined above with a soluble alkali or alkaline earth metal base, which base is soluble in dry aprotic solvent at a temperature from -100 to 50°C and then reacting a β-lactam of the formula: 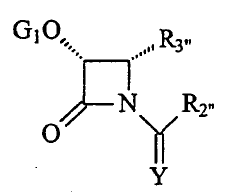 in which
Y is defined above: G₁ represents a hydroxyl protecting group:
R_{2"} represents a radical R₂ defined above or a protected R₂ whenever R₂ includes one or more active hydrogens:
R_{3"} represents a radical R₃ defined above or a protected R₃ whenever R₃ includes one or more active hydrogens:
with the baccatin III derivative.

2. A process according to claim 1, in which R₂ represents a radical RO-, RS-, or RR'N- in which R represents a straight chain or branched alkyl radical containing 1 to 10 carbon atoms, a straight chain or branched alkenyl radical containing 2 to 10 carbon atoms, or a straight chain or branched alkynyl radical containing 2 to 10 carbon atoms, a cycloalkyl radical containing 3 to 10 carbon atoms, a heterocycloalkyl radical containing 3 to 10 carbon atoms, a cycloalkenyl radical containing 3 to 10 carbon atoms, a heterocycloalkenyl radical containing 3 to 10 carbon atoms, a polycycloalkyl radical containing 6 to 20 carbon atoms, an aryl radical containing 6 to 20 carbons, a heteroaryl radical containing 3 to 15 carbon atoms; these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms; R' is hydrogen or R defined above; R and R' can form a cyclic structure which contains 2-10 carbon atoms;
R₃ represents a straight chain or branched alkyl radical containing 1 to 10 carbon atoms, a straight chain or branched alkenyl radical containing 2 to 10 carbon atoms, or a straight chain or branched alkynyl radical containing 2 to 10 carbon atoms, a cycloalkyl radical containing 3 to 10 carbon atoms, a cycloalkenyl radical containing 3 to 10 carbon atoms, a polycycloalkyl radical containing 6 to 20 carbon atoms, an aryl radical containing 6 to 10 carbon atoms; these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals, the alkyl portion of which contains 1 to 15 carbon atoms, aryloxycarbonyl, the aryl portion of which contains 6 to 20 carbon atoms, or heteroaryloxycarbonyl the heteroaryl portion of which contains 3 to 15 carbon atoms;
R_{2"} represents a radical R₂ defined above or a protected R₂ whenever R₂ includes one or more active hydrogens;
R_{3"} represents a radical R₃ defined above or a protected R₃ whenever R₃ includes one or more active hydrogens.

3. A process according to claim 1, wherein R₂ represents an RO-, RS-, or RR'N- in which R is an unsubstituted or substituted radical selected from methyl, ethyl, propyl, isopropyl, butyl, isobutyl, tert-butyl, pentyl, isopentyl, neopentyl, hexyl,
isohexyl, heptyl, isoheptyl, octyl, isooctyl, cyclopropyl, cyclobutyl, cyclopentyl, cyclohexyl, cycloheptyl, cyclooctyl, adamantyl, vinyl, allyl, phenyl, naphthyl, furyl, pyrrolyl, pyridyl, cyclopentenyl, cyclohexenyl, cycloheptenyl, oxiranyl, tetrahydrofuryl, pyrrolidinyl, piperidinyl, tetrahydropyranyl, dihydrofuryl, dihydropyrrolyl, dihydropyranyl, dihydropyridyl; R is hydrogen or R defined above; cyclic RR'N- radical is aziridino, azetidino, pyrrolidino, piperidino or morpholino group;
R₃ is an unsubstituted or substituted radical selected from methyl, ethyl, propyl, isopropyl, butyl, isobutyl, tert-butyl, pentyl, isopentyl, neopentyl, hexyl, isohexyl, heptyl, isoheptyl, octyl, isooctyl, cyclohexylmethyl, cyclohexylethyl, benzyl, phenylethyl, cyclopropyl, cyclobutyl, cyclopentyl, cyclohexyl, cycloheptyl, cyclooctyl, adamantyl, vinyl, allyl, ethynyl, propargyl, phenyl, naphthyl, cyclopentenyl, cyclohexenyl and cycloheptenyl;
R_{2"} represents a radical R₂ defined above or a protected R₂ wherever R₂ includes one or more active hydrogens;
R_{3"} represents a radical R₃ defined above or a protected R₃ wherever R₃ includes one or more active hydrogens;
G₁ represents a group protecting the hydroxyl function selected from methoxylmethyl (MOM), methoxyethyl (MEM), 1-ethoxyethyl (EE), benzyloxymethyl, (β-trimethylsilyl-ethoxyl)-methyl, tetrahydropyranyl, 2,2,2-trichloroethoxylcarbonyl (Troc), benzyloxycarbonyl (CBZ), tert-butoxycarbonyl (t-BOC), 9-fluorenylmethoxycarbonyl (Fmoc), 2,2,2-trichloroethoxymethyl, trimethylsilyl, triethylsilyl, tripropylsilyl, dimethylethylsilyl, dimethyl (t-butyl)silyl, diethylinethylsilyl, dimethylphenylsilyl and diphenylmethylsilyl, acetyl, chloroacetyl, dichloroacetyl, trichloroacetyl and trifluoroacetyl;
G₂ represents an acetyl or a 2,2,2-trichloroethoxycarbonyl (Troc) group;
G₃ represents a 2,2,2-trichloroethoxycarbonyl (Troc) or silyl group selected from trimethylsilyl, triethylsilyl, tripropylsilyl, dimethylethylsilyl, dimethylphenylsilyl, dimethyl(t-butyl)silyl, diethylmethylsilyl and diphenylmethylsilyl.

4. A process according to claim 1, 2 or 3 wherein M is an alkali metal.

5. A process according to claim 4 wherein M is am alkali metal selected from lithium, sodium and potassium.

6. A process according to claim 4, wherein M is sodium or potassium.

7. A process according to any one of claims 1 to 6 wherein the base is sodium hexamethyldisilazide, potassium hexamethyldisilazide, lithium hexamethyldisilazide, potassium diisopropylamide, sodium diisopropylamide or lithium diisopropylamide.

8. A process according to any one of the preceding claims wherein R₁ is a hydrogen, an acetyl or a trichloroethoxycarbonyl (Troc); R₄ is a hydrogen, a triethylsilyl or a trichloroethoxycarbonyl (Troc); R₅ is a hydrogen, a triethylsilyl or ethoxyethyl.

9. The process according to any one of claims 1 to 7 wherein R₂ represents RO- in which R is a methyl, ethyl, propyl, isopropyl, butyl, isobutyl, tert-butyl, neopentyl, cyclohexyl, phenyl, benzyl or 9-fluoroenylmethyl; R₃ is phenyl, tolyl, 4-methoxyphenyl, 3,4-dimethoxyphenyl, 4-fluorophenyl, 4-trifluoromethylphenyl, 1-naphthyl, 2-naphthyl or 2-phenylethenyl; R₅ is a hydrogen.

10. The process according to any one of claims 1 to 7 wherein R₂ is a methylamino, ethylamino, propylamino, isopropylamino, butylamino, isobutylamino, tert-butylamino, neopentylamino, cyclohexylamino, phenylamino or benzylamino, dimethylamino or morpholino group; R₅ is a hydrogen.

11. The process according to claim 1 wherein R₁ is a hydrogen or an acetyl; R₂ (=R₂") is tert-butoxy or tert-butylamino; R₃ (=R₃") is a phenyl; Y is oxygen; R₄ is a hydrogen; R₅ is a hydrogen; G₁ is an ethoxyethyl, triethylsilyl or trichloroethoxycarbonyl (Troc); M is sodium or potassium.

## Claims (Claims for the following Contracting State(s): AT, BE, CH, DE, DK, ES, FR, GB, GR, IE, IT, LI, LU, MC, NL, SE)

1. A process for the preparation of a taxane derivative of the formula: 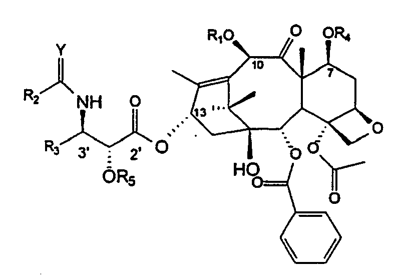 in which
R₁ represents a hydrogen or an acyl or an alkyl or an alkenyl or an alkynyl or a carbocyclic aryl or a heteroaryl radical or a hydroxyl protecting group;
R₂ represents an RO-, RS- or RR'N- in which R represents a straight chain or branched alkyl, alkenyl or alkynyl, cycloalkyl, heterocycloalkyl, cycloalkenyl, heterocycloalkenyl, carbocyclic aryl or heteroaryl radical, these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals, the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms; R' is hydrogen or R defined above; R and R' can be connected to form a cyclic structure;
Y is oxygen or sulfur;
R₃ represents a straight chain or branched alkyl, alkenyl or alkynyl, cycloalkyl, cycloalkenyl or carbocyclic aryl radical, these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl radicals, the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms;
R₄ represents hydrogen or an acyl radical or an unsubstituted straight chain or branched alkyl, alkenyl or alkynyl radical, an unsubstituted cycloalkyl, heterocycloalkyl, cycloalkenyl or heterocycloalkenyl radical, an unsubstituted aryl or heteroaryl radical, or a hydroxyl group protecting group;
R₅ represents hydrogen or an acyl radical or an unsubstituted straight chain or branched alkyl, alkenyl, or alkynyl radical, an unsubstituted cycloalkyl, heterocycloalkyl, cycloalkenyl or heterocycloalkenyl radical, an unsubstituted aryl or heteroaryl radical, or a hydroxyl protecting group;
which process comprises preparing a baccatin III derivative of the formula: 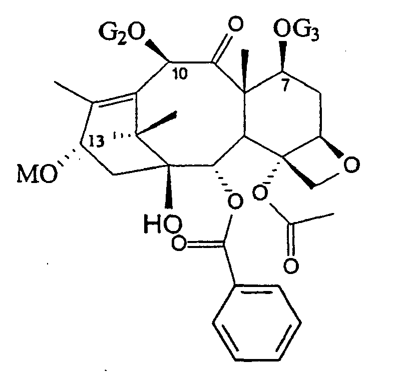 in which M is an alkali metal atom (ion);
G₂ represents a hydroxyl protecting group or an acyl radical or an unsubstituted straight chain or branched alkyl, alkenyl, or alkynyl radical, an unsubstituted cycloalkyl, heterocycloalkyl, cycloalkenyl or heterocycloalkenyl radical, or an unsubstituted aryl or heteroaryl radical; and
G₃ represents a hydroxyl protecting group or an acyl radical or an unsubstituted straight chain or branched alkyl, alkenyl or alkynyl radical, an unsubstituted cycloalkyl, heterocycloalkyl, cycloalkenyl, or heterocycloalkenyl radical, or an unsubstituted aryl or heteroaryl radical,
by reacting a protected baccatin III of formula: 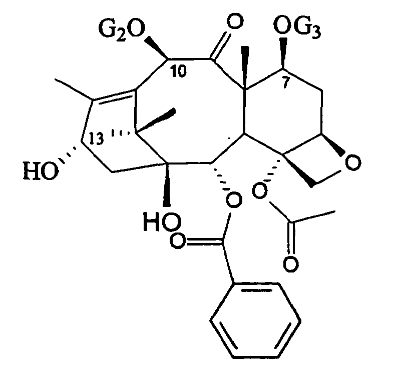 in which G₂ and G₃ are as defined above with a soluble alkali metal base which base is sodium hexamethyldisilazide, potassium hexamethyldisilazide, lithium hexamethyldisilazide, sodium diisopropylamide or potassium diisopropylamide, and then reacting a β-lactam of the formula: 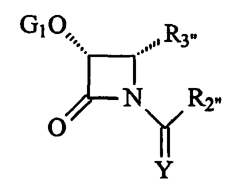 in which
Y is defined above: G₁ represents a hydroxyl protecting group:
R_{2"} represents a radical R₂ defined above or a protected R₂ whenever R₂ includes one or more active hydrogens:
R_{3"} represents a radical R₃ defined above or a protected R₃ whenever R₃ includes one or more active hydrogens:
with the baccatin III derivative.

2. A process according to claim 1, in which R₂ represents a radical RO-, RS-, or RR'N- in which R represents a straight chain or branched alkyl radical containing 1 to 10 carbon atoms, a straight chain or branched alkenyl radical containing 2 to 10 carbon atoms, or a straight chain or branched alkynyl radical containing 2 to 10 carbon atoms, a cycloalkyl radical containing 3 to 10 carbon atoms, a heterocycloalkyl radical containing 3 to 10 carbon atoms, a cycloalkenyl radical containing 3 to 10 carbon atoms, a heterocycloallcenyl radical containing 3 to 10 carbon atoms, a polycycloalkyl radical containing 6 to 20 carbon atoms, an aryl radical containing 6 to 20 carbons, a heteroaryl radical containing 3 to 15 carbon atoms; these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals the alkyl portion of which contains 1 to 15 carbon atoms, aryloxycarbonyl the aryl portion of which contains 6 to 20 carbon atoms, or heteroaryloxycarbonyl the heteroaryl portion of which contains 3 to 15 carbon atoms; R' is hydrogen or R defined above; R and R' can form a cyclic structure which contains 2-10 carbon atoms;
R₃ represents a straight chain or branched alkyl radical containing 1 to 10 carbon atoms, a straight chain or branched alkenyl radical containing 2 to 10 carbon atoms, or a straight chain or branched alkynyl radical containing 2 to 10 carbon atoms, a cycloalkyl radical containing 3 to 10 carbon atoms, a cycloalkenyl radical containing 3 to 10 carbon atoms, a polycycloalkyl radical containing 6 to 20 carbon atoms, an aryl radical containing 6 to 10 carbon atoms; these radicals being optionally substituted with one or more halogen, hydroxyl, alkoxy, aryloxy, heteroaryloxy, amino, alkylamino, dialkylamino, mercapto, alkylthio, arylthio, heteroarylthio, cyano, carboxyl and alkoxycarbonyl radicals, the alkyl portion of which contains 1 to 15 carbon atoms, aryloxycarbonyl, the aryl portion of which contains 6 to 20 carbon atoms, or heteroaryloxycarbonyl the heteroaryl portion of which contains 3 to 15 carbon atoms;
R_{2"} represents a radical R₂ defined above or a protected R₂ whenever R₂ includes one or more active hydrogens;
R_{3"} represents a radical R₃ defined above or a protected R₃ whenever R₃ includes one or more active hydrogens.

3. A process according to claim 1, wherein R₂ represents an RO-, RS-, or RR'N- in which R is an unsubstituted or substituted radical selected from methyl, ethyl, propyl, isopropyl, butyl, isobutyl, tert-butyl, pentyl, isopentyl, neopentyl, hexyl, isohexyl, heptyl, isoheptyl, octyl, isooctyl, cyclopropyl, cyclobutyl, cyclopentyl, cyclohexyl, cycloheptyl, cyclooctyl, adamantyl, vinyl, allyl, phenyl, naphthyl, furyl, pyrrolyl, pyridyl, cyclopentenyl, cyclohexenyl, cycloheptenyl, oxiranyl, tetrahydrofuryl, pyrrolidinyl, piperidinyl, tetrahydropyranyl, dihydrofuryl, dihydropyrrolyl, dihydropyranyl, dihydropyridyl; R is hydrogen or R defined above; cyclic RR'N- radical is aziridino, azetidino, pyrrolidino, piperidino or morpholino group;
R₃ is an unsubstituted or substituted radical selected from methyl, ethyl, propyl, isopropyl, butyl, isobutyl, tert-butyl, pentyl, isopentyl, neopentyl, hexyl, isohexyl, heptyl, isoheptyl, octyl, isooctyl, cyclohexylmethyl, cyclohexylethyl, benzyl, phenylethyl, cyclopropyl, cyclobutyl, cyclopentyl, cyclohexyl, cycloheptyl, cyclooctyl, adamantyl, vinyl, allyl, ethynyl, propargyl, phenyl, naphthyl, cyclopentenyl, cyclohexenyl and cycloheptenyl;
R_{2"} represents a radical R₂ defined above or a protected R₂ wherever R₂ includes one or more active hydrogens;
R_{3"} represents a radical R₃ defined above or a protected R₃ wherever R₃ includes one or more active hydrogens;
G₁ represents a group protecting the hydroxyl function selected from methoxylmethyl (MOM), methoxyethyl (MEM), 1-ethoxyethyl (EE), benzyloxymethyl, (β-trimethylsilyl-ethoxyl)-methyl, tetrahydropyranyl, 2,2,2-trichloroethoxylcarbonyl (Troc), benzyloxycarbonyl (CBZ), tert-butoxycarbonyl (t-BOC), 9-fluorenylmethoxycarbonyl (Fmoc), 2,2,2-trichloroethoxymethyl, trimethylsilyl, triethylsilyl, tripropylsilyl, dimethylethylsilyl, dimethyl (t-butyl)silyl, diethylmethylsilyl, dimethylphenylsilyl and diphenylmethylsilyl, acetyl, chloroacetyl, dichloroacetyl, trichloroacetyl and trifluoroacetyl;
G₂ represents an acetyl or a 2,2,2-trichloroethoxycarbonyl (Troc) group;
G₃ represents a 2,2,2-trichloroethoxycarbonyl (Troc) or silyl group selected from trimethylsilyl, triethylsilyl, tripropylsilyl, dimethylethylsilyl, dimethylphenylsilyl, dimethyl(t-butyl)silyl, diethylmethylsilyl and diphenylmethylsilyl.

4. A process according to claim 1, 2 or 3, wherein M is sodium or potassium.

5. A process according to claim 1, 2 or 3 wherein R₁ is a hydrogen, an acetyl or a trichloroethoxycarbonyl (Troc); R₄ is a hydrogen, a triethylsilyl or a trichloroethoxycarbonyl (Troc); R₅ is a hydrogen, a triethylsilyl or ethoxyethyl.

6. The process according to claim1, 2 or 3 wherein R₂ represents RO- in which R is a methyl, ethyl, propyl, isopropyl, butyl, isobutyl, tert-butyl, neopentyl, cyclohexyl, phenyl, benzyl or 9-fluoroenylmethyl; R₃ is phenyl, tolyl, 4-methoxyphenyl, 3,4-dimethoxyphenyl, 4-fluorophenyl, 4-trifluoromethylphenyl, 1-naphthyl, 2-naphthyl or 2-phenylethenyl; R₅ is a hydrogen.

7. The process according to claim 1, 2 or 3 wherein R₂ is a methylamino, ethylamino, propylamino, isopropylamino, butylamino, isobutylamino, tert-butylamino, neopentylamino, cyclohexylamino, phenylamino or benzylamino, dimethylamino or morpholino group; R₅ is a hydrogen.

8. The process according to any one of the preceding claims wherein R₁ is a hydrogen or an acetyl; R₂ (=R₂") is tert-butoxy or tert-butylamino; R₃ (=R₃") is a phenyl; Y is oxygen; R₄ is a hydrogen; R₅ is a hydrogen; G₁ is an ethoxyethyl, triethylsilyl or trichloroethoxycarbonyl (Troc); M is sodium or potassium.

## Patentansprüche (Patentansprüche für folgende(n) Vertragsstaat(en): PT)

1. Verfahren zur Herstellung eines Taxanderivats der Formel: 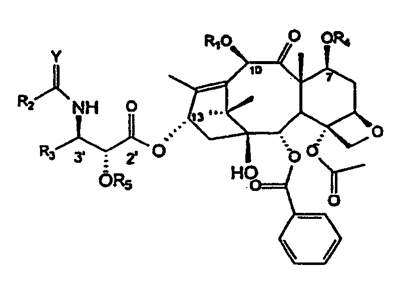 wobei
R₁ für einen Wasserstoff oder ein Acyl oder ein Alkyl oder ein Alkenyl oder ein Alkynyl oder ein carbocyclisches Aryl oder ein Heteroarylradikal oder eine Hydroxylschutzgruppe steht;
R₂ für ein RO-, RS- oder RR'N- steht, worin R für ein geradkettiges oder verzweigtes Alkyl-, Alkenyl- oder Alkynyl-, Cycloalkyl-, Heterocycloalkyl-, Cycloalkenyl-, Heterocycloalkenyl-, carbocyclisches Aryl- oder Heteroarylradikal steht, wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält; R' Wasserstoff oder das vorstehend definierte R ist; R und R' zur Bildung einer zyklischen Struktur verbunden werden können;
Y Sauerstoff oder Schwefel ist;
R₃ für ein geradkettiges oder verzweigtes Alkyl-, Alkenyl- oder Alkynyl-, Cycloalkyl-, Cycloalkenyl- oder carbocyclisches Arylradikal steht, wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält;
R₄ für Wasserstoff oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal, ein unsubstituiertes Aryl- oder Heteroarylradikal oder eine Hydroxylgruppenschutzgruppe steht;
R₅ für Wasserstoff oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal, ein unsubstituiertes Aryl- oder Heteroarylradikal oder eine Hydroxylschutzgruppe steht;
welches Verfahren die Herstellung eines Baccatin-III-Derivats der Formel: 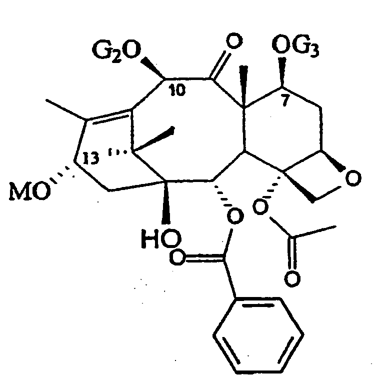 umfasst, worin M ein Alkalimetall- oder Alkalinerdmetallatom (Ion) ist;
G₂ für eine Hydroxylschutzgruppe oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal oder ein unsubstituiertes Aryl- oder Heteroarylradikal steht; und
G₃ für eine Hydroxylschutzgruppe oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal oder ein unsubstituiertes Aryl- oder Heteroarylradikal steht;
indem ein geschütztes Baccatin III der Formel: 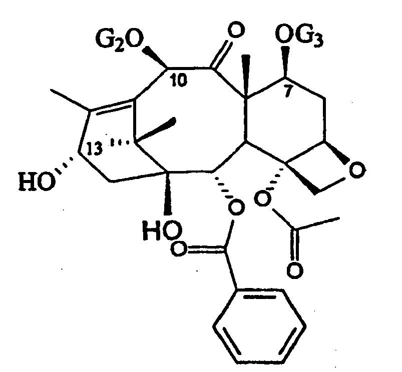 in dem G₂ und G₃ wie vorstehend definiert sind, mit einer löslichen Alkali- oder Alkalinerdmetallbase umgesetzt wird, welche Base in einem trockenen aprotischen Lösungsmittel bei einer Temperatur von -100 bis 50°C löslich ist, und anschließend ein β-Lactam der Formel: 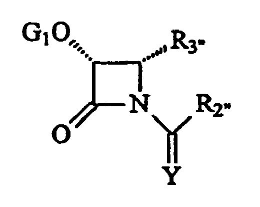 worin
Y wie vorstehend definiert ist; G₁ für eine Hydroxylschutzgruppe steht;
R_{2"} für ein vorstehend definiertes Radikal R₂ oder ein geschütztes R₂ steht, wann immer R₂ einen oder mehrere aktive Wasserstoffe aufweist;
R_{3"} für ein vorstehend definiertes Radikal R₃ oder ein geschütztes R₃ steht, wann immer R₃ einen oder mehrere aktive Wasserstoffe aufweist;
mit dem Baccatin-III-Derivat umgesetzt wird.

2. Verfahren nach Anspruch 1, wobei R₂ für ein radikal RO-, RS- oder RR'N-steht, worin R für ein geradkettiges oder verzweigtes Alkylradikal mit 1 bis 10 Kohlenstoffatomen, ein geradkettiges oder verzweigtes Alkenylradikal mit 2 bis 10 Kohlenstoffatomen oder ein geradkettiges oder verzweigtes Alkynylradikal mit 2 bis 10 Kohlenstoffatomen, ein Cycloalkylradikal mit 3 bis 10 Kohlenstoffatomen, ein Heterocycloalkylradikal mit 3 bis 10 Kohlenstoffatomen, ein Cyloalkenylradikal mit 3 bis 10 Kohlenstoffatomen, ein Heterocycloalkenylradikal mit 3 bis 10 Kohlenstoffatomen, ein Polycycloalkylradikal mit 6 bis 20 Kohlenstoffatomen, ein Arylradikal mit 6 bis 20 Kohlenstoffatomen, ein Heteroarylradikal mit 3 bis 15 Kohlenstoffatomen steht; wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält; R' Wasserstoff oder das vorstehend definierte R ist; R und R' eine cyclische Struktur bilden können, die 2-10 Kohlenstoffatome enthält;
R₃ für ein geradkettiges oder verzweigtes Alkylradikal mit 1 bis 10 Kohlenstoffatomen, ein geradkettiges oder verzweigtes Alkenylradikal mit 2 bis 10 Kohlenstoffatomen oder ein geradkettiges oder verzweigtes Alkynylradikal mit 2 bis 10 Kohlenstoffatomen, ein Cycloalkylradikal mit 3 bis 10 Kohlenstoffatomen, ein Cycloalkenylradikal mit 3 bis 10 Kohlenstoffen, ein Polycycloalkylradikal mit 6 bis 20 Kohlenstoffatomen, ein Arylradikal mit 6 bis 10 Kohlenstoffatomen steht; wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält;
R_{2"} für ein vorstehend definiertes Radikal R₂ oder ein geschütztes R₂ steht, wann immer R₂ einen oder mehrere aktive Wasserstoffe aufweist;
R_{3"} für ein vorstehend definiertes Radikal R₃ oder ein geschütztes R₃ steht, wann immer R₃ einen oder mehrere aktive Wasserstoffe aufweist.

3. Verfahren nach Anspruch 1, wobei R₂ für ein RO-, RS- oder RR'N- steht, worin R ein unsubstituiertes oder substituieres Radikal ist, das ausgewählt ist aus Methyl, Ethyl, Propyl, Isopropyl, Butyl, Isobutyl, tert-Butyl, Pentyl, Isopentyl, Neopentyl, Hexyl, Isohexyl, Heptyl, Isoheptyl, Octyl, Isooctyl, Cyclopropyl, Cyclobutyl, Cyclopentyl, Cyclohexyl, Cycloheptyl, Cyclooctyl, Adamantyl, Vinyl, Allyl, Phenyl, Napththyl, Furyl, Pyrrolyl, Pyridyl, Cyclopentenyl, Cyclohexenyl, Cycloheptenyl, Oxiranyl, Tetrahydrofuryl, Pyrrolidinyl, Piperidinyl, Tetrahydropyranyl, Dihydrofuryl, Dihydropyrrolyl, Dihydropyranyl, Dihydropyridyl; R' Wasserstoff oder das vorstehend definierte R ist; das cyclische RR'N-Radikal eine Aziridin-, Azetidin-, Pyrrolidin-, Piperidin- oder Morpholingruppe ist;
R₃ ein unsubstituiertes oder substituiertes Radikal ist, ausgewählt aus Methyl, Ethyl, Propyl, Isopropyl, Butyl, Isobutyl, tert-Butyl, Pentyl, Isopentyl, Neopentyl, Hexyl, Isohexyl, Heptyl, Isoheptyl, Octyl, Isooctyl, Cyclohexylmethyl, Cyclohexylethyl, Benzyl, Phenylethyl, Cyclopropyl, Cyclobutyl, Cyclopentyl, Cyclohexyl, Cycloheptyl, Cyclooctyl, Adamantyl, Vinyl, Allyl, Ethynyl, Propargyl, Phenyl, Napththyl, Cyclopentenyl, Cyclohexenyl und Cycloheptenyl;
R_{2"} für ein vorstehend definiertes Radikal R₂ oder ein geschütztes R₂ steht, wann immer R₂ einen oder mehrere aktive Wasserstoffe aufweist;
R_{3"} für ein vorstehend definiertes Radikal R₃ oder ein geschütztes R₃ steht, wann immer R₃ einen oder mehrere aktive Wasserstoffe aufweist;
G₁ für eine die Hydroxylfunktion schützende Gruppe steht, die aus Methoxylmethyl (MOM), Methoxyethyl (MEM), 1-Ethoxyethyl (EE), Benzyloxymethyl, (β-Trimethylsilylethoxy)-methyl, Tetrahydropyranyl, 2,2,2-Trichlorethoxycarbonyl (Troc), Benzyloxycarbonyl (CBZ), tert-Butoxycarbonyl (t-BOC), 9-Fluorenylmethoxycarbonyl (Fmoc), 2,2,2-Trichlorethoxymethyl, Trimethylsilyl, Triethylsilyl, Tripropylsilyl, Dimethylethylsilyl, Dimethyl(t-butyl)silyl, Diethylmethylsilyl, Dimethylphenylsilyl und Diphenylmethylsilyl, Acetyl, Chloracetyl, Dichloracetyl, Trichloracetyl und Trifluoracetyl ausgewählt ist;
G₂ für eine Acetyl- oder eine 2,2,2-Trichlorethoxycarbonyl (Troc)-Gruppe steht;
G₃ für eine 2,2,2-Trichlorethoxycarbonyl (Troc)- oder Silylgruppe steht, die aus Trimethylsilyl, Triethylsilyl, Tripropylsilyl, Dimethylethylsilyl, Dimethylphenylsilyl, Dimethyl(t-butyl)silyl, Diethylmethylsilyl und Diphenylmethylsilyl ausgewählt ist.

4. Verfahren nach Anspruch 1, 2 oder 3, wobei M ein Alkalimetall ist.

5. Verfahren nach Anspruch 4, wobei M ein aus Lithium, Natrium und Kalium ausgewähltes Alkalimetall ist.

6. Verfahren nach Anspruch 4, wobei M Natrium oder Kalium ist.

7. Verfahren nach einem der Ansprüche 1 bis 6, wobei die Base Natriumhexamethyldisilazid, Kaliumhexamethyldisilazid, Lithiumhexamethyldisilazid, Kaliumdiisopropylamid, Natriumdüsopropylamid oder Lithiumdiisopropylamid ist.

8. Verfahren nach einem der vorhergehenden Ansprüche, wobei R₁ ein Wasserstoff, ein Acetyl oder ein Trichlorethoxycarbonyl (Troc) ist; R₄ ein Wasserstoff, ein Triethylsilyl oder ein Trichlorethoxycarbonyl (Troc) ist; R₅ ein Wasserstoff, ein Triethylsilyl oder Ethoxyethyl ist.

9. Verfahren nach einem der Ansprüche 1 bis 7, wobei R₂ für RO- steht, worin R ein Methyl, Ethyl, Propyl, Isopropyl, Butyl, Isobutyl, tert-Butyl, Neopentyl, Cyclohexyl, Phenyl, Benzyl oder 9-Fluorenylmethyl ist; R₃ Phenyl, Tolyl, 4-Methoxyphenyl, 3,4-Dimethoxyphenyl, 4-Fluorphenyl, 4-Trifluormethylphenyl, 1-Naphthyl, 2-Naphthyl oder 2-Phenylethenyl ist; R₅ ein Wasserstoff ist.

10. Verfahren nach einem der Ansprüche 1 bis 7, wobei R₂ eine Methylamin-, Ethylamin-, Propylamin-, Isopropylamin-, Butylamin-, Isobutylamin-, tert-Butylamin-, Neopentylamin-, Cyclohexylamin-, Phenylamin- oder Benzylamin-, Dimethylamin- oder Morpholingruppe ist; R₅ ein Wasserstoff ist.

11. Verfahren nach Anspruch 1, wobei R₁ ein Wasserstoff oder ein Acetyl ist; R₂ (=R₂") tert-Butoxy oder tert-Butylamin ist; R₃ (=R₃") ein Phenyl ist; Y Sauerstoff ist; R₄ ein Wasserstoff ist; R₅ ein Wasserstoff ist; G₁ ein Ethoxyethyl, Triethylsilyl oder Trichlorethoxycarbonyl (Troc) ist; M Natrium oder Kalium ist.

## Patentansprüche (Patentansprüche für folgende(n) Vertragsstaat(en): AT, BE, CH, DE, DK, ES, FR, GB, GR, IE, IT, LI, LU, MC, NL, SE)

1. Verfahren zur Herstellung eines Taxanderivats der Formel: 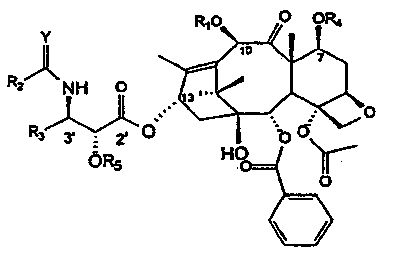 wobei
R₁ für einen Wasserstoff oder ein Acyl oder ein Alkyl oder ein Alkenyl oder ein Alkynyl oder ein carbocyclisches Aryl oder ein Heteroarylradikal oder eine Hydroxylschutzgruppe steht;
R₂ für ein RO-, RS- oder RR'N- steht, worin R für ein geradkettiges oder verzweigtes Alkyl-, Alkenyl- oder Alkynyl-, Cycloalkyl-, Heterocycloalkyl-, Cycloalkenyl-, Heterocycloalkenyl-, carbocyclisches Aryl- oder Heteroarylradikal steht, wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält; R' Wasserstoff oder das vorstehend definierte R ist; R und R' zur Bildung einer zyklischen Struktur verbunden werden können;
Y Sauerstoff oder Schwefel ist;
R₃ für ein geradkettiges oder verzweigtes Alkyl-, Alkenyl- oder Alkynyl-, Cycloalkyl-, Cycloalkenyl- oder carbocyclisches Arylradikal steht, wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält;
R₄ für Wasserstoff oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal, ein unsubstituiertes Aryl- oder Heteroarylradikal oder eine Hydroxylgruppenschutzgruppe steht;
R₅ für Wasserstoff oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal, ein unsubstituiertes Aryl- oder Heteroarylradikal oder eine Hydroxylschutzgruppe steht;
welches Verfahren die Herstellung eines Baccatin-III-Derivats der Formel: 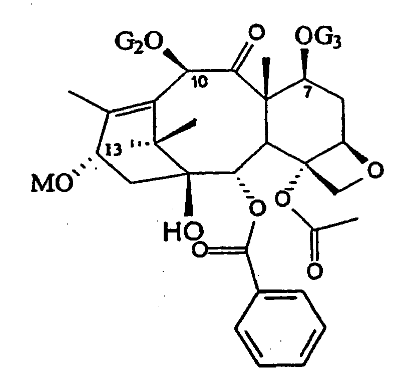 umfasst, worin M ein Alkalimetallatom (Ion) ist;
G₂ für eine Hydroxylschutzgruppe oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal oder ein unsubstituiertes Aryl- oder Heteroarylradikal steht; und
G₃ für eine Hydroxylschutzgruppe oder ein Acylradikal oder ein unsubstituiertes geradkettiges oder verzweigtes Alkyl-, Alkenyl- oder Alkynylradikal, ein unsubstituiertes Cycloalkyl-, Heterocycloalkyl-, Cycloalkenyl- oder Heterocycloalkenylradikal oder ein unsubstituiertes Aryl- oder Heteroarylradikal steht;
indem ein geschütztes Baccatin III der Formel: 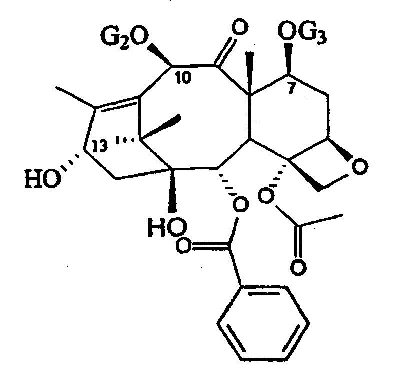 in dem G₂ und G₃ wie vorstehend definiert sind, mit einer löslichen Alkalimetallbase umgesetzt wird, welche Base Natriumhexamethyldisilazid, Kaliumhexamethyldisilazid, Lithiumhexamethyldisilazid, Natriumdiisopropylamid oder Kaliumdiisopropylamid ist, und anschließend ein β-Lactam der Formel: 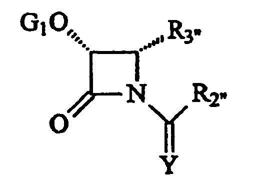 worin
Y wie vorstehend definiert ist; G₁ für eine Hydroxylschutzgruppe steht:;
R_{2"} für ein vorstehend definiertes Radikal R₂ oder ein geschütztes R₂ steht, wann immer R₂ einen oder mehrere aktive Wasserstoffe aufweist;
R_{3"} für ein vorstehend definiertes Radikal R₃ oder ein geschütztes R₃ steht, wann immer R₃ einen oder mehrere aktive Wasserstoffe aufweist;
mit dem Baccatin-III-Derivat umgesetzt wird.

2. Verfahren nach Anspruch 1, wobei R₂ für ein radikal RO-, RS- oder RR'N-steht, worin R für ein geradkettiges oder verzweigtes Alkylradikal mit 1 bis 10 Kohlenstoffatomen, ein geradkettiges oder verzweigtes Alkenylradikal mit 2 bis 10 Kohlenstoffatomen oder ein geradkettiges oder verzweigtes Alkynylradikal mit 2 bis 10 Kohlenstoffatomen, ein Cycloalkylradikal mit 3 bis 10 Kohlenstoffatomen, ein Heterocycloalkylradikal mit 3 bis 10 Kohlenstoffatomen, ein Cyloalkenylradikal mit 3 bis 10 Kohlenstoffatomen, ein Heterocycloalkenylradikal mit 3 bis 10 Kohlenstoffatomen, ein Polycycloalkylradikal mit 6 bis 20 Kohlenstoffatomen, ein Arylradikal mit 6 bis 20 Kohlenstoffatomen, ein Heteroarylradikal mit 3 bis 15 Kohlenstoffatomen steht; wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält; R' Wasserstoff oder das vorstehend definierte R ist; R und R' eine cyclische Struktur bilden können, die 2-10 Kohlenstoffatome enthält;
R₃ für ein geradkettiges oder verzweigtes Alkylradikal mit 1 bis 10 Kohlenstoffatomen, ein geradkettiges oder verzweigtes Alkenylradikal mit 2 bis 10 Kohlenstoffatomen oder ein geradkettiges oder verzweigtes Alkynylradikal mit 2 bis 10 Kohlenstoffatomen, ein Cycloalkylradikal mit 3 bis 10 Kohlenstoffatomen, ein Cycloalkenylradikal mit 3 bis 10 Kohlenstoffen, ein Polycycloalkylradikal mit 6 bis 20 Kohlenstoffatomen, ein Arylradikal mit 6 bis 10 Kohlenstoffatomen steht; wobei diese Radikale gegebenenfalls substituiert werden durch ein oder mehrere Halogen-, Hydroxyl-, Alkoxy-, Aryloxy-, Heteroaryloxy-, Amino-, Alkylamino-, Dialkylamino-, Mercapto-, Alkylthio-, Arylthio-, Heteroarylthio-, Cyano-, Carboxyl- und Alkoxycarbonylradikale, deren Alkylabschnitt 1 bis 15 Kohlenstoffatome enthält, Aryloxycarbonyl, dessen Arylabschnitt 6 bis 20 Kohlenstoffatome enthält, oder Heteroaryloxycarbonyl, dessen Heteroarylabschnitt 3 bis 15 Kohlenstoffatome enthält;
R_{2"} für ein vorstehend definiertes Radikal R₂ oder ein geschütztes R₂ steht, wann immer R₂ einen oder mehrere aktive Wasserstoffe aufweist;
R_{3"} für ein vorstehend definiertes Radikal R₃ oder ein geschütztes R₃ steht, wann immer R₃ einen oder mehrere aktive Wasserstoffe aufweist.

3. Verfahren nach Anspruch 1, wobei R₂ für ein RO-, RS- oder RR'N- steht, worin R ein unsubstituiertes oder substituieres Radikal ist, das ausgewählt ist aus Methyl, Ethyl, Propyl, Isopropyl, Butyl, Isobutyl, tert-Butyl, Pentyl, Isopentyl, Neopentyl, Hexyl, Isohexyl, Heptyl, Isoheptyl, Octyl, Isooctyl, Cyclopropyl, Cyclobutyl, Cyclopentyl, Cyclohexyl, Cycloheptyl, Cyclooctyl, Adamantyl, Vinyl, Allyl, Phenyl, Napththyl, Furyl, Pyrrolyl, Pyridyl, Cyclopentenyl, Cyclohexenyl, Cycloheptenyl, Oxiranyl, Tetrahydrofuryl, Pyrrolidinyl, Piperidinyl, Tetrahydropyranyl, Dihydrofuryl, Dihydropyrrolyl, Dihydropyranyl, Dihydropyridyl; R' Wasserstoff oder das vorstehend definierte R ist; das cyclische RR'N-Radikal eine Aziridin-, Azetidin-, Pyrrolidin-, Piperidin- oder Morpholingruppe ist;
R₃ ein unsubstituiertes oder substituiertes Radikal ist, ausgewählt aus Methyl, Ethyl, Propyl, Isopropyl, Butyl, Isobutyl, tert-Butyl, Pentyl, Isopentyl, Neopentyl, Hexyl, Isohexyl, Heptyl, Isoheptyl, Octyl, Isooctyl, Cyclohexylmethyl, Cyclohexylethyl, Benzyl, Phenylethyl, Cyclopropyl, Cyclobutyl, Cyclopentyl, Cyclohexyl, Cycloheptyl, Cyclooctyl, Adamantyl, Vinyl, Allyl, Ethynyl, Propargyl, Phenyl, Napththyl, Cyclopentenyl, Cyclohexenyl und Cycloheptenyl;
R_{2"} für ein vorstehend definiertes Radikal R₂ oder ein geschütztes R₂ steht, wann immer R₂ einen oder mehrere aktive Wasserstoffe aufweist;
R_{3"} für ein vorstehend definiertes Radikal R₃ oder ein geschütztes R₃ steht, wann immer R₃ einen oder mehrere aktive Wasserstoffe aufweist;
G₁ für eine die Hydroxylfunktion schützende Gruppe steht, die aus Methoxylmethyl (MOM), Methoxyethyl (MEM), 1-Ethoxyethyl (EE), Benzyloxymethyl, (β-Trimethylsilylethoxy)-methyl, Tetrahydropyranyl, 2,2,2-Trichlorethoxycarbonyl (Troc), Benzyloxycarbonyl (CBZ), tert-Butoxycarbonyl (t-BOC), 9-Fluorenylmethoxycarbonyl (Fmoc), 2,2,2-Trichlorethoxymethyl, Trimethylsilyl, Triethylsilyl, Tripropylsilyl, Dimethylethylsilyl, Dimethylsilyl(t-butyl)silyl, Diethylmethylsilyl, Dimethylphenylsilyl und Diphenylmethyl, Acetyl, Chloracetyl, Dichloracetyl, Trichloracetyl und Trifluoracetyl ausgewählt ist;
G₂ für eine Acetyl- oder eine 2,2,2-Trichlorethoxycarbonyl (Troc)-Gruppe steht;
G₃ für eine 2,2,2-Trichlorethoxycarbonyl (Troc)- oder Silylgruppe steht, die aus Trimethylsilyl, Triethylsilyl, Tripropylsilyl, Dimethylethylsilyl, Dimethylphenylsilyl, Dimethyl(t-butyl)silyl, Diethylmethylsilyl und Diphenylmethylsilyl ausgewählt ist.

4. Verfahren nach Anspruch 1, 2 oder 3, wobei M Natrium oder Kalium ist.

5. Verfahren nach Anspruch 1, 2 oder 3, wobei R₁ ein Wasserstoff, ein Acetyl oder ein Trichlorethoxycarbonyl (Troc) ist; R₄ ein Wasserstoff, ein Triethylsilyl oder ein Trichlorethoxycarbonyl (Troc) ist; R₅ ein Wasserstoff, ein Triethylsilyl oder Ethoxyethyl ist.

6. Verfahren nach Anspruch 1, 2 oder 3, wobei R₂ für RO- steht, worin R ein Methyl, Ethyl, Propyl, Isopropyl, Butyl, Isobutyl, tert-Butyl, Neopentyl, Cyclohexyl, Phenyl, Benzyl oder 9-Fluorenylmethyl ist; R₃ Phenyl, Tolyl, 4-Methoxyphenyl, 3,4-Dimethoxyphenyl, 4-Fluorphenyl, 4-Trifluormethylphenyl, 1-Naphthyl, 2-Naphthyl oder 2-Phenylethenyl ist; R₅ ein Wasserstoff ist.

7. Verfahren nach Anspruch 1, 2 oder 3, wobei R₂ eine Methylamin-, Ethylamin-, Propylamin-, Isopropylamin-, Butylamin-, Isobutylamin-, tert-Butylamin-, Neopentylamin-, Cyclohexylamin-, Phenylamin- oder Benzylamin-, Dimethylamin- oder Morpholingruppe ist; R₅ ein Wasserstoff ist.

8. Verfahren nach einem der vorhergehenden Ansprüche, wobei R₁ ein Wasserstoff oder ein Acetyl ist; R₂ (=R₂") ein tert-Butoxy oder tert-Butylamin ist; R₃ (=R₃") ein Phenyl ist; Y Sauerstoff ist; R₄ ein Wasserstoff ist; R₅ ein Wasserstoff ist; G₁ ein Ethoxyethyl, Triethylsilyl oder Trichlorethoxycarbonyl (Troc) ist; M Natrium oder Kalium ist.

## Revendications (Revendications pour l'(les) Etat(s) contractant(s) suivant(s): PT)

1. Procédé pour la préparation d'un dérivé de taxane de formule : 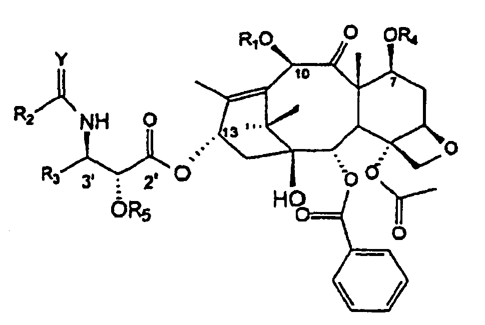 dans laquelle
R₁ représente un hydrogène ou un radical acyle ou alkyle ou alcényle ou alcynyle ou aryle carbocyclique ou hétéroaryle ou un groupe protecteur d'hydroxyle ;
R₂ représente un radical RO-, RS- ou RR'N- dans lequel R représente un radical alkyle, alcényle ou alcynyle à chaîne linéaire ou ramifiée, cycloalkyle, hétérocycloalkyle, cycloalcényle, hétérocycloalcényle, aryle carbocyclique ou hétéroaryle, ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles et alcoxycarbonyles, dont la partie alkyle contient 1 à 15 atomes de carbone, aryloxycarbonyles dont la partie aryle contient 6 à 20 atomes de carbone ou hétéroaryloxycarbonyles dont la partie hétéroaryle contient 3 à 15 atomes de carbone ; R' est un hydrogène ou R défini ci-dessus ; R et R' peuvent être reliés pour former une structure cyclique ;
Y est un oxygène ou un soufre ;
R₃ représente un radical alkyle, alcényle ou alcynyle à chaîne linéaire ou ramifiée, cycloalkyle, cycloalcényle ou aryle carbocyclique, ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles, alcoxycarbonyles dont la partie alkyle contient 1 à 15 atomes de carbone, aryloxycarbonyles dont la partie aryle contient 6 à 20 atomes de carbone ou hétéroaryloxycarbonyles dont la partie hétéroaryle contient 3 à 15 atomes de carbone ;
R₄ représente un hydrogène ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué, un radical aryle ou hétéroaryle non substitué ou un groupe protecteur de groupe hydroxyle ;
R₅ représente un hydrogène ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué, un radical aryle ou hétéroaryle non substitué ou un groupe protecteur d'hydroxyle ;
lequel procédé comprend de préparer un dérivé de la baccatine III de formule : 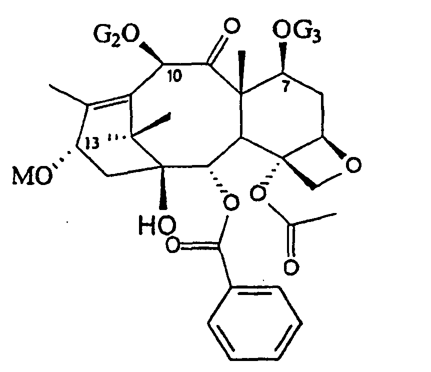 dans laquelle M est un atome (ion) de métal alcalin ou de métal alcalinoterreux ;
G₂ représente un groupe protecteur d'hydroxyle ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué ou un radical aryle ou hétéroaryle non substitué ; et
G₃ représente un groupe protecteur d'hydroxyle ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué ou un radical aryle ou hétéroaryle non substitué,
en faisant réagir une baccatine III protégée de formule : 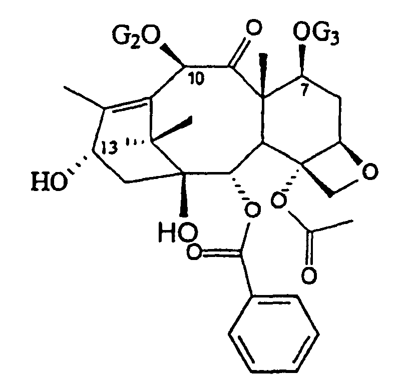 dans laquelle G₂ et G₃ sont tels que définis ci-dessus avec une base de métal alcalin ou alcalinoterreux soluble, laquelle base est soluble dans un solvant aprotique sec à une température allant de -100 à 50°C et en faisant ensuite réagir un β-lactame de formule : 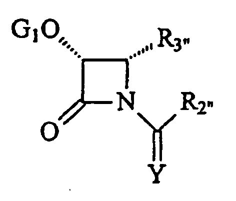 dans laquelle
Y est défini ci-dessus ; G₁ représente un groupe protecteur d'hydroxyle ;
R_{2"} représente un radical R₂ défini ci-dessus ou un R₂ protégé quand R₂ comprend un ou plusieurs hydrogènes actifs ;
R_{3"} représente un radical R₃ défini ci-dessus ou un R₃ protégé quand R₃ comprend un ou plusieurs hydrogènes actifs ;
avec le dérivé de la baccatine III.

2. Procédé selon la revendication 1, dans lequel R₂ représente un radical RO-, RS- ou RR'N- dans lequel R représente un radical alkyle à chaîne linéaire ou ramifiée contenant 1 à 10 atomes de carbone, un radical alcényle à chaîne linéaire ou ramifiée contenant 2 à 10 atomes de carbone ou un radical alcynyle à chaîne linéaire ou ramifiée contenant 2 à 10 atomes de carbone, un radical cycloalkyle contenant 3 à 10 atomes de carbone, un radical hétérocycloalkyle contenant 3 à 10 atomes de carbone, un radical cycloalcényle contenant 3 à 10 atomes de carbone, un radical hétérocycloalcényle contenant 3 à 10 atomes de carbone, un radical polycycloalkyle contenant 6 à 20 atomes de carbone, un radical aryle contenant 6 à 20 atomes de carbone, un radical hétéroaryle contenant 3 à 15 atomes de carbone ; ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles et alcoxycarbonyles dont la partie alkyle contient 1 à 15 atomes de carbone, aryloxycarbonyles dont la partie aryle contient 6 à 20 atomes de carbone ou hétéroaryloxycarbonyles dont la partie hétéroaryle contient 3 à 15 atomes de carbone ; R' est un hydrogène ou R défini ci-dessus ; R et R' peuvent former une structure cyclique qui contient 2-10 atomes de carbone ;
R₃ représente un radical alkyle à chaîne linéaire ou ramifiée contenant 1 à 10 atomes de carbone, un radical alcényle à chaîne linéaire ou ramifiée contenant 2 à 10 atomes de carbone ou un radical alcynyle à chaîne linéaire ou ramifiée contenant 2 à 10 atomes de carbone, un radical cycloalkyle contenant 3 à 10 atomes de carbone, un radical cycloalcényle contenant 3 à 10 atomes de carbone, un radical polycycloalkyle contenant 6 à 20 atomes de carbone, un radical aryle contenant 6 à 10 atomes de carbone ; ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles et alcoxycarbonyles, dont la partie alkyle contient 1 à 15 atomes de carbone, aryloxycarbonyles, dont la partie aryle contient 6 à 20 atomes de carbone ou hétéroaryloxycarbonyles dont la partie hétéroaryle contient 3 à 15 atomes de carbone ;
R_{2"} représente un radical R₂ défini ci-dessus ou un R₂ protégé quand R₂ comprend un ou plusieurs hydrogènes actifs ;
R_{3"} représente un radical R₃ défini ci-dessus ou un R₃ protégé quand R₃ comprend un ou plusieurs hydrogènes actifs.

3. Procédé selon la revendication 1, dans lequel R₂ représente un radical RO-, RS- ou RR'N- dans lequel R est un radical substitué ou non substitué choisi entre un méthyle, éthyle, propyle, isopropyle, butyle, isobutyle, *tert*-butyle, pentyle, isopentyle, néopentyle, hexyle, isohexyle, heptyle, isoheptyle, octyle, isooctyle, cyclopropyle, cyclobutyle, cyclopentyle, cyclohexyle, cycloheptyle, cyclooctyle, adamantyle, vinyle, allyle, phényle, naphtyle, furyle, pyrrolyle, pyridyle, cyclopentényle, cyclohexényle, cycloheptényle, oxiranyle, tétrahydrofuryle, pyrrolidinyle, pipéridinyle, tétrahydropyranyle, dihydrofuryle, dihydropyrrolyle, dihydropyranyle, dihydropyridyle ; R' est un hydrogène ou R défini ci-dessus ; le radical RR'N- cyclique est un groupe aziridino, azétidino, pyrrolidino, pipéridino ou morpholino ;
R₃ est un radical substitué ou non substitué choisi entre un méthyle, éthyle, propyle, isopropyle, butyle, isobutyle, *tert*-butyle, pentyle, isopentyle, néopentyle, hexyle, isohexyle, heptyle, isoheptyle, octyle, isooctyle, cyclohexylméthyle, cyclohexyléthyle, benzyle, phényléthyle, cyclopropyle, cyclobutyle, cyclopentyle, cyclohexyle, cycloheptyle, cyclooctyle, adamantyle, vinyle, allyle, éthynyle, propargyle, phényle, naphtyle, cyclopentényle, cyclohexényle et cycloheptényle ;
R_{2"} représente un radical R₂ défini ci-dessus ou un R₂ protégé quand R₂ comprend un ou plusieurs hydrogènes actifs ;
R_{3"} représente un radical R₃ défini ci-dessus ou un R₃ protégé quand R₃ comprend un ou plusieurs hydrogènes actifs ;
G₁ représente un groupe protégeant la fonction hydroxyle choisi entre un méthoxyméthyle (MOM), méthoxyéthyle (MEM), 1-éthoxyéthyle (EE), benzyloxyméthyle, (β-triméthylsilyléthoxyl)méthyle, tétrahydropyranyle, 2,2,2-trichloroéthoxycarbonyle (Troc), benzyloxycarbonyle (CBZ), *tert*-butoxycarbonyle (t-BOC), 9-fluorénylméthoxycarbonyle (Fmoc), 2,2,2-trichloroéthoxyméthyle, triméthylsilyle, triéthylsilyle, tripropylsilyle, diméthyléthylsilyle, diméthyl(*t-*butyl)silyle, diéthylméthylsilyle, diméthylphénylsilyle et diphénylméthylsilyle, acétyle, chloroacétyle, dichloroacétyle, trichloroacétyle et trifluoroacétyle ;
G₂ représente un acétyle ou un groupe 2,2,2-trichloroéthoxycarbonyle (Troc) ;
G₃ représente un 2,2,2-trichloroéthoxycarbonyle (Troc) ou un groupe silyle choisi entre un triméthylsilyle, triéthylsilyle, tripropylsilyle, diméthyléthylsilyle, diméthylphénylsilyle, diméthyl(*t*-butyl)silyle, diéthylméthylsilyle et diphénylméthylsilyle.

4. Procédé selon la revendication 1, 2 ou 3 dans lequel M est un métal alcalin.

5. Procédé selon la revendication 4 dans lequel M est un métal alcalin choisi entre le lithium, le sodium et le potassium.

6. Procédé selon la revendication 4, dans lequel M est le sodium ou le potassium.

7. Procédé selon l'une quelconque des revendications 1 à 6 dans lequel la base est le bis(triméthylsilyl)amidure de sodium, le bis(triméthylsilyl)amidure de potassium, le bis(triméthylsilyl)amidure de lithium, le diisopropylamidure de potassium, le diisopropylamidure de sodium ou le diisopropylamidure de lithium.

8. Procédé selon l'une quelconque des revendications précédentes dans lequel R₁ est un hydrogène, un acétyle ou un trichloroéthoxycarbonyle (Troc) ; R₄ est un hydrogène, un triéthylsilyle ou un trichloroéthoxycarbonyle (Troc) ; R₅ est un hydrogène , un triéthylsilyle ou un éthoxyéthyle.

9. Procédé selon l'une quelconque des revendications 1 à 7 dans lequel R₂ représente RO- où R est un méthyle, éthyle, propyle, isopropyle, butyle, isobutyle, *tert*-butyle, néopentyle, cyclohexyle, phényle, benzyle ou 9-fluorénylméthyle ; R₃ est un phényle, tolyle, 4-méthoxyphényle, 3,4-diméthoxyphényle, 4-fluorophényle, 4-trifluorométhylphényle, 1-naphtyle, 2-naphtyle ou 2-phényléthényle ; R₅ est un hydrogène.

10. Procédé selon l'une quelconque des revendications 1 à 7 dans lequel R₂ est un groupe méthylamino, éthylamino, propylamino, isopropylamino, butylamino, isobutylamino, *tert*-butylamino, néopentylamino, cyclohexylamino, phénylamino ou benzylamino, diméthylamino ou morpholino ; R₅ est un hydrogène.

11. Procédé selon la revendication 1 dans lequel R₁ est un hydrogène ou un acétyle ; R₂ (=R_{2"}) est un *tert*-butoxy ou *tert*-butylamino ; R₃ (=R_{3"}) est un phényle ; Y est un oxygène ; R₄ est un hydrogène ; R₅ est un hydrogène ; G₁ est un éthoxyéthyle, triéthylsilyle ou trichloroéthoxycarbonyle (Troc) ; M est le sodium ou le potassium.

## Revendications (Revendications pour l'(les) Etat(s) contractant(s) suivant(s): AT, BE, CH, DE, DK, ES, FR, GB, GR, IE, IT, LI, LU, MC, NL, SE)

1. Procédé pour la préparation d'un dérivé de taxane de formule : 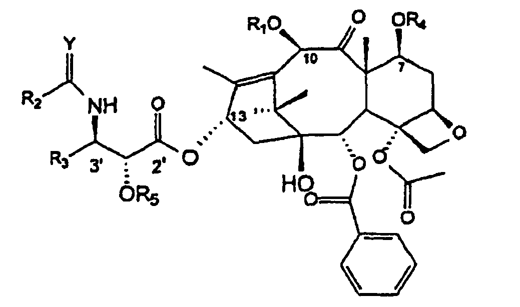 dans laquelle
R₁ représente un hydrogène ou un radical acyle ou alkyle ou alcényle ou alcynyle ou aryle carbocyclique ou hétéroaryle ou un groupe protecteur d'hydroxyle ;
R₂ représente un radical RO-, RS- ou RR'N- dans lequel R représente un radical alkyle, alcényle ou alcynyle à chaîne linéaire ou ramifiée, cycloalkyle, hétérocycloalkyle, cycloalcényle, hétérocycloalcényle, aryle carbocyclique ou hétéroaryle, ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles et alcoxycarbonyles, dont la partie alkyle contient 1 à 15 atomes de carbone, aryloxycarbonyles dont la partie aryle contient 6 à 20 atomes de carbone ou hétéroaryloxycarbonyles dont la partie hétéroaryle contient 3 à 15 atomes de carbone ; R' est un hydrogène ou R défini ci-dessus ; R et R' peuvent être reliés pour former une structure cyclique ;
Y est un oxygène ou un soufre ;
R₃ représente un radical alkyle, alcényle ou alcynyle à chaîne linéaire ou ramifiée, cycloalkyle, cycloalcényle ou aryle carbocyclique, ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles, alcoxycarbonyles dont la partie alkyle contient 1 à 15 atomes de carbone, aryloxycarbonyle dont la partie aryle contient 6 à 20 atomes de carbone ou hétéroaryloxycarbonyle dont la partie hétéroaryle contient 3 à 15 atomes de carbone ;
R₄ représente un hydrogène ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué, un radical aryle ou hétéroaryle non substitué ou un groupe protecteur de groupe hydroxyle ;
R₅ représente un hydrogène ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué, un radical aryle ou hétéroaryle non substitué ou un groupe protecteur d'hydroxyle ;
lequel procédé comprend de préparer un dérivé de la baccatine III de formule : 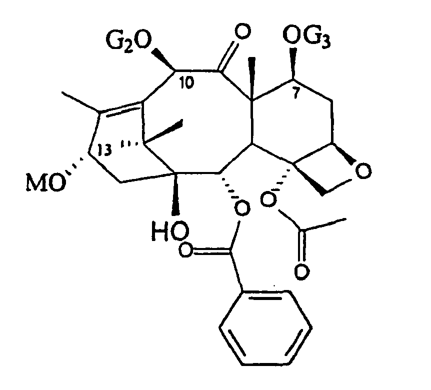 dans laquelle M est un atome (ion) de métal alcalin ;
G₂ représente un groupe protecteur d'hydroxyle ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué ou un radical aryle ou hétéroaryle non substitué ; et
G₃ représente un groupe protecteur d'hydroxyle ou un radical acyle ou un radical alkyle, alcényle ou alcynyle à chaîne linéaire ou ramifiée non substitué, un radical cycloalkyle, hétérocycloalkyle, cycloalcényle ou hétérocycloalcényle non substitué ou un radical aryle ou hétéroaryle non substitué,
en faisant réagir une baccatine III protégée de formule : 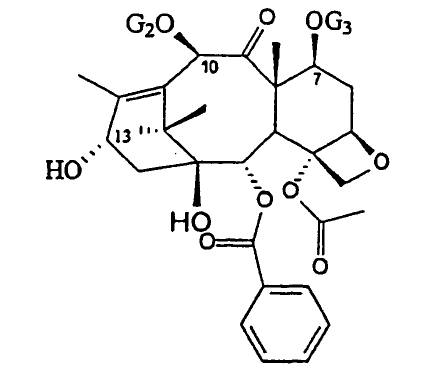 dans laquelle G₂ et G₃ sont tels que définis ci-dessus avec une base de métal alcalin soluble laquelle base est le bis(triméthylsilyl)amidure de sodium, le bis(triméthylsilyl)amidure de potassium, le bis(triméthylsilyl)amidure de lithium, le diisopropylamidure de sodium ou le diisopropylamidure de potassium et en faisant ensuite réagir un β-lactame de formule : 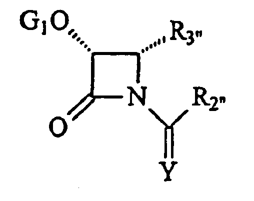 dans laquelle
Y est défini ci-dessus ; G₁ représente un groupe protecteur d'hydroxyle ;
R_{2"} représente un radical R₂ défini ci-dessus ou un R₂ protégé quand R₂ comprend un ou plusieurs hydrogènes actifs ;
R_{3"} représente un radical R₃ défini ci-dessus ou un R₃ protégé quand R₃ comprend un ou plusieurs hydrogènes actifs ;
avec le dérivé de la baccatine III.

2. Procédé selon la revendication 1, dans lequel R₂ représente un radical RO-, RS- ou RR'N- dans lequel R représente un radical alkyle à chaîne linéaire ou ramifiée contenant 1 à 10 atomes de carbone, un radical alcényle à chaîne linéaire ou ramifiée contenant 2 à 10 atomes de carbone ou un radical alcynyle à chaîne linéaire ou ramifiée contenant 2 à 10 atomes de carbone, un radical cycloalkyle contenant 3 à 10 atomes de carbone, un radical hétérocycloalkyle contenant 3 à 10 atomes de carbone, un radical cycloalcényle contenant 3 à 10 atomes de carbone, un radical hétérocycloalcényle contenant 3 à 10 atomes de carbone, un radical polycycloalkyle contenant 6 à 20 atomes de carbone, un radical aryle contenant 6 à 20 atomes de carbone, un radical hétéroaryle contenant 3 à 15 atomes de carbone ; ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles et alcoxycarbonyles dont la partie alkyle contient 1 à 15 atomes de carbone, aryloxycarbonyles dont la partie aryle contient 6 à 20 atomes de carbone ou hétéroaryloxycarbonyles dont la partie hétéroaryle contient 3 à 15 atomes de carbone ; R' est un hydrogène ou R défini ci-dessus ; R et R' peuvent former une structure cyclique qui contient 2-10 atomes de carbone ;
R₃ représente un radical alkyle à chaîne linéaire ou ramifiée contenant 1 à 10 atomes de carbone, un radical alcényle à chaîne linéaire ou ramifiée contenant 2 à 10 atomes de carbone ou un radical alcynyle à chaîne linéaire ou ramifiée contenant 2 à 10 atomes de carbone, un radical cycloalkyle contenant 3 à 10 atomes de carbone, un radical cycloalcényle contenant 3 à 10 atomes de carbone, un radical polycycloalkyle contenant 6 à 20 atomes de carbone, un radical aryle contenant 6 à 10 atomes de carbone ; ces radicaux étant facultativement substitués par un ou plusieurs halogènes, hydroxyles, radicaux alcoxy, aryloxy, hétéroaryloxy, amino, alkylamino, dialkylamino, mercapto, alkylthio, arylthio, hétéroarylthio, cyano, carboxyles et alcoxycarbonyles, dont la partie alkyle contient 1 à 15 atomes de carbone, aryloxycarbonyles, dont la partie aryle contient 6 à 20 atomes de carbone ou hétéroaryloxycarbonyles dont la partie hétéroaryle contient 3 à 15 atomes de carbone ;
R_{2"} représente un radical R₂ défini ci-dessus ou un R₂ protégé quand R₂ comprend un ou plusieurs hydrogènes actifs ;
R_{3"} représente un radical R₃ défini ci-dessus ou un R₃ protégé quand R₃ comprend un ou plusieurs hydrogènes actifs.

3. Procédé selon la revendication 1, dans lequel R₂ représente un radical RO-, RS- ou RR'N- dans lequel R est un radical substitué ou non substitué choisi entre un méthyle, éthyle, propyle, isopropyle, butyle, isobutyle, *tert*-butyle, pentyle, isopentyle, néopentyle, hexyle, isohexyle, heptyle, isoheptyle, octyle, isooctyle, cyclopropyle, cyclobutyle, cyclopentyle, cyclohexyle, cycloheptyle, cyclooctyle, adamantyle, vinyle, allyle, phényle, naphtyle, furyle, pyrrolyle, pyridyle, cyclopentényle, cyclohexényle, cycloheptényle, oxiranyle, tétrahydrofuryle, pyrrolidinyle, pipéridinyle, tétrahydropyranyle, dihydrofuryle, dihydropyrrolyle, dihydropyranyle, dihydropyridyle ; R' est un hydrogène ou R défini ci-dessus ; le radical RR'N- cyclique est un groupe aziridino, azétidino, pyrrolidino, pipéridino ou morpholino ;
R₃ est un radical substitué ou non substitué choisi entre un méthyle, éthyle, propyle, isopropyle, butyle, isobutyle, *tert*-butyle, pentyle, isopentyle, néopentyle, hexyle, isohexyle, heptyle, isoheptyle, octyle, isooctyle, cyclohexylméthyle, cyclohexyléthyle, benzyle, phényléthyle, cyclopropyle, cyclobutyle, cyclopentyle, cyclohexyle, cycloheptyle, cyclooctyle, adamantyle, vinyle, allyle, éthynyle, propargyle, phényle, naphtyle, cyclopentényle, cyclohexényle et cycloheptényle ;
R_{2"} représente un radical R₂ défini ci-dessus ou un R₂ protégé quand R₂ comprend un ou plusieurs hydrogènes actifs ;
R_{3"} représente un radical R₃ défini ci-dessus ou un R₃ protégé quand R₃ comprend un ou plusieurs hydrogènes actifs ;
G₁ représente un groupe protégeant la fonction hydroxyle choisi entre un méthoxyméthyle (MOM), méthoxyéthyle (MEM), 1-éthoxyéthyle (EE), benzyloxyméthyle, (β-triméthylsilyléthoxyl)méthyle, tétrahydropyranyle, 2,2,2-trichloroéthoxycarbonyle (Troc), benzyloxycarbonyle (CBZ), *tert*-butoxycarbonyle (t-BOC), 9-fluorénylméthoxycarbonyle (Fmoc), 2,2,2-trichloroéthoxyméthyle, triméthylsilyle, triéthylsilyle, tripropylsilyle, diméthyléthylsilyle, diméthyl(*t-*butyl)silyle, diéthylméthylsilyle, diméthylphénylsilyle et diphénylméthylsilyle, acétyle, chloroacétyle, dichloroacétyle, trichloroacétyle et trifluoroacétyle ;
G₂ représente un acétyle ou un groupe 2,2,2-trichloroéthoxycarbonyle (Troc) ;
G₃ représente un 2,2,2-trichloroéthoxycarbonyle (Troc) ou un groupe silyle choisi entre un triméthylsilyle, triéthylsilyle, tripropylsilyle, diméthyléthylsilyle, diméthylphénylsilyle, diméthyl(*t*-butyl)silyle, diéthylméthylsilyle et diphénylméthylsilyle.

4. Procédé selon la revendication 1, 2 ou 3 dans lequel M est le sodium ou le potassium.

5. Procédé selon la revendication 1, 2 ou 3 dans lequel R₁ est un hydrogène, un acétyle ou un trichloroéthoxycarbonyle (Troc) ; R₄ est un hydrogène, un triéthylsilyle ou un trichloroéthoxycarbonyle (Troc) ; R₅ est un hydrogène , un triéthylsilyle ou un éthoxyéthyle.

6. Procédé selon la revendication 1, 2 ou 3 dans lequel R₂ représente RO- où R est un méthyle, éthyle, propyle, isopropyle, butyle, isobutyle, *tert*-butyle, néopentyle, cyclohexyle, phényle, benzyle ou 9-fluorénylméthyle ; R₃ est un phényle, tolyle, 4-méthoxyphényle, 3,4-diméthoxyphényle, 4-fluorophényle, 4-trifluorométhylphényle, 1-naphtyle, 2-naphtyle ou 2-phényléthényle ; R₅ est un hydrogène.

7. Procédé selon la revendication 1, 2 ou 3 dans lequel R₂ est un groupe méthylamino, éthylamino, propylamino, isopropylamino, butylamino, isobutylamino, *tert*-butylamino, néopentylamino, cyclohexylamino, phénylamino ou benzylamino, diméthylamino ou morpholino ; R₅ est un hydrogène.

8. Procédé selon l'une quelconque des revendications précédentes dans lequel R₁ est un hydrogène ou un acétyle ; R₂ (=R_{2"}) est un *tert-*butoxy ou *tert*-butylamino ; R₃ (=R_{3"}) est un phényle ; Y est un oxygène ; R₄ est un hydrogène ; R₅ est un hydrogène ; G₁ est un éthoxyéthyle, triéthylsilyle ou trichloroéthoxycarbonyle (Troc) ; M est le sodium ou le potassium.
